# EUROPEAN PATENT APPLICATION

(11) **EP 4 501 311 A1**
(43) Date of publication of application: **05.02.2025**
(21) Application number: 23779910.1
(22) Date of filing: 22.03.2023
(51) Int. Cl.: A61K 8/891, A61Q 1/06, A61Q 1/10, A61Q 1/14, A61Q 17/04

(54) **COSMETIC**

(30) Priority: 31.03.2022 JP 2022059395
(71) Applicant: SHIN-ETSU CHEMICAL CO., LTD., Tokyo 1000005 (JP)
(72) Inventor: KANAI Tomoya, Annaka-shi, Gunma 379-0224 (JP); KIKUCHI Hiroko, Tokyo 100-0005 (JP)
(74) Representative: Mewburn Ellis LLP
(86) International application number: PCT/JP2023/011108
(87) International publication number: WO 2023/189896

(57) **Abstract**

This cosmetic contains at least one branched organopolysiloxane represented by

(R¹₃SiO_{1/2})ₐ(R¹₂SiO_{2/2})_{b}(R¹SiO_{3/2})_{c}(SiO_{4/2})_{d}

(In the formula, R¹s are independently a hydrogen atom, a hydroxyl group, or a monovalent alkyl group having 1-3 carbon atoms; and two or more of R¹s are monovalent alkyl groups having 2 or 3 carbon atoms.)
having a boiling point of 87-119°C at 10 hPa. The branched organopolysiloxane provides a cosmetic which: has a light feel and good spreadability; has a uniform cosmetic film and high water repellency and can thus yield a uniform film, does not have a strong oily feeling; can provide a good feeling of use; and has excellent storage stability and long-lasting cosmetic properties (cosmetic retentivity) even when an oily component such as silicone and an organic ultraviolet absorber are mixed.

## Description

### TECHNICAL FIELD

The present invention relates to a cosmetic containing a branched organopolysiloxane.

### BACKGROUND ART

Silicones, typified by dimethylpolysiloxanes, are frequently used as oils in cosmetics in recent years to benefit from their characteristics, such as light feel, good spreadability, excellent water repellency, and high safety.

For example, cosmetics contain volatile cyclic siloxanes, such as octamethyltetrasiloxane (D4), decamethylpentasiloxane (D5), and dodecamethylhexasiloxane (D6), linear siloxanes with a kinematic viscosity at 25°C of 0.65 to 6 mm²/s, and volatile siloxanes with branched siloxane chains, such as tristrimethylsiloxymethylsilane (M3T). Such cosmetics excel in light feel, spreadability, and water repellency when applied to the skin (Patent Document 1).

However, these silicones sometimes exhibit low affinity for oil ingredients, such as waxes, that are added to solidify cosmetics. Such low affinity inhibits the crystallization of the oil ingredient to destroy the uniformity of the cosmetic that is obtained or to invite a failure to obtain the desired hardness of the preparation in some cases. Furthermore, the above silicones are poorly compatible with polar oils and may fail to give transparent mixtures or may impair the feeling on use and the stability of the cosmetics. This tendency is particularly noticeable when an organic ultraviolet absorber is used.

On the other hand, it is known that compatibilizers, such as phenyl-modified silicones or esters, are used in order to increase the compatibility with polar oils, waxes, and the like. In this case, however, the light feel of silicone is sometimes lost and the cosmetics may have a heavy feeling on use. Furthermore, many of these oils are nonvolatile or low-volatile and therefore the cosmetics may give a strong oily feel when applied to the skin.

On the other hand, Patent Document 2 proposes a sunscreen composition that contains ingredients including an organic ultraviolet absorber and a volatile oil. The formulation involves, for example, a highly oil-absorptive powder in order to reduce the stickiness of the composition. However, a study of compatibility enhancement is limited to the combined use of a volatile silicone oil and a hydrocarbon oil, such as isododecane. High loading of such a low-viscosity hydrocarbon oil sometimes causes skin irritation or other problems. Furthermore, the powder, when added in a large amount, makes it difficult to apply the cosmetic uniformly and the cosmetic film is often insufficient in durability.

### PRIOR ART DOCUMENTS

### PATENT DOCUMENTS

Patent Document 1: WO 01/015658
Patent Document 2: JP-A 2018-70556

### SUMMARY OF INVENTION

### TECHNICAL PROBLEM

The present invention has been made in view of the circumstances discussed above. It is therefore an object of the present invention to provide a cosmetic containing a branched organopolysiloxane that has a light feel and good spreadability, can form a uniform cosmetic film having high water repellency, uniformity, and a moderately oily feel, has a good feeling on use, and attains excellent storage stability and cosmetic durability (long-lasting cosmetic properties) even when an oil ingredient, such as silicone, and an organic ultraviolet absorber are added.

### SOLUTION TO PROBLEM

The present inventors carried out intensive studies directed to achieving the above object and have found that the problems described above can be solved by adding to a cosmetic a branched organopolysiloxane with a specific structure that has a boiling point in a predetermined range. Based on the finding, the present inventors have completed the present invention.

Thus, the present invention provides a cosmetic.
1. A cosmetic including one or more branched organopolysiloxanes represented by formula (1) below:

   (R¹₃SiO_{1/2})ₐ(R¹₂SiO_{2/2})_{b}(R¹SiO_{3/2})_{c}(SiO_{4/2})_{d} (1)

   (wherein R¹ is independently is a hydrogen atom, a hydroxyl group, or a C1-C3 monovalent alkyl group, a is an integer of 0 to 5, b is an integer of 0 to 3, c is an integer of 0 to 2, d is 0 or 1, c+ d is 1 or 2, and a + b + c + d is an integer of 4 to 7, two or more R¹ are 2 or C3 monovalent alkyl group),
   the branched organopolysiloxanes having a boiling point at 10 hPa of 87 to 119°C.
2. The cosmetic according to 1, wherein the formula (1) has b = 0, d = 0, and c = 1.
3. The cosmetic according to 1 or 2, wherein the branched organopolysiloxane is one or more selected from 1,5-diethyl-3-trimethylsiloxy-1,1,3,5,5-pentamethyltrisiloxane and 1,5 -diethyl-3 -ethyldimethylsiloxy-1,1 ,3,5,5-pentamethyltrisiloxane.
4. The cosmetic according to any one of 1 to 3, further including an oil ingredient that is solid at 25°C.
5. The cosmetic according to 4, wherein the oil ingredient that is solid at 25°C is one or more selected from polyethylene, ceresin, ozokerite, candelilla wax, beeswax, microcrystalline wax, stearyl alcohol, behenyl alcohol, and cetanol.
6. The cosmetic according to any one of 1 to 5, further including an organic ultraviolet absorber.
7. The cosmetic according to 6, wherein the organic ultraviolet absorber is one or more selected from ethylhexyl methoxycinnamate, hexyl diethylaminohydroxybenzoylbenzoate, ethylhexyl salicylate, polysilicone-15, t-butylmethoxydibenzoylmethane, oxybenzone, methylenebisbenzotriazolyltetramethylbutylphenol, bisethylhexyloxyphenolmethoxyphenyltriazine, and octocrylene.

### ADVANTAGEOUS EFFECTS OF INVENTION

The cosmetic containing a branched organopolysiloxane according to the present invention has a light feel and good spreadability, can form a uniform cosmetic film having high water repellency, uniformity, and a moderately oily feel, has a good feeling on use, and attains excellent over-time stability and cosmetic durability even when an oil ingredient, such as silicone, and an organic ultraviolet absorber are added.

### BRIEF DESCRIPTION OF DRAWINGS

[FIG. 1] FIG. 1 is a ¹H-NMR spectrum chart of a branched organopolysiloxane synthesized in Synthesis Example 1.
[FIG. 2] FIG. 2 is a ²⁹Si-NMR spectrum chart of the branched organopolysiloxane synthesized in Synthesis Example 1.

### DESCRIPTION OF EMBODIMENTS

The present invention will be described in detail below. The present invention is not limited to the following description. The compound names given in the present invention are sometimes those cited in *Kesho̅hin Hyo̅ji Meisho̅* [Japanese Cosmetic Labeling Names] or the International Nomenclature of Cosmetic Ingredients (INCI). In this Specification, when the name of an ingredient differs in these two lists, the *Kesho̅hin Hyo̅ji Meisho̅* name is given first in lower case letters, followed by, in parenthesis, the capitalized INCI name. When the name from the two lists is the same, it is followed simply by "(INCI)".

### I. Branched Organopolysiloxanes

The organopolysiloxanes of the present invention are represented by formula (1) below:

(R¹₃SiO_{1/2})ₐ(R¹₂SiO_{2/2})_{b}(R¹SiO_{3/2})_{c}(SiO_{4/2})_{d} (1)

(wherein R¹ is independently is a hydrogen atom, a hydroxyl group, or a C1-C3 monovalent alkyl group, a is an integer of 0 to 5, b is an integer of 0 to 3, c is an integer of 0 to 2, d is 0 or 1, c + d is 1 or 2, and a + b + c + d is an integer of 4 to 7, two or more R¹ are 2 or C3 monovalent alkyl group). The branched organopolysiloxanes have a boiling point at 10 hPa of 87 to 119°C. The organopolysiloxanes may be used singly, or two or more may be used in combination.

R¹ independently at each occurrence is a hydrogen atom, a hydroxyl group, or a C1-C3 monovalent alkyl group. Specific examples include methyl group, ethyl group, and propyl group. R¹ is a C2 or C3 monovalent alkyl group, specifically, may be an ethyl group or a propyl group at two or more occurrences. If R¹s are all methyl groups or if only one R¹ is a C2 or C3 monovalent alkyl group, the compound does not show sufficient affinity for an oil ingredient that is solid at 25°C or a polar oil, and the cosmetic that is obtained will not attain excellent over-time stability and cosmetic durability.

The letter a is an integer of 0 to 5, b is an integer of 0 to 3, c is an integer of 0 to 2, d is 0 or 1, c + d is 1 or 2, and a + b + c + d is an integer of 4 to 7. In particular, it is preferable that b = 0, d = 0, and c = 1. Such a branched organopolysiloxane does not produce a strong oily feel when added to a cosmetic and offers a good feeling on use. In addition, the branched organopolysiloxane allows the cosmetic to attain excellent over-time stability and cosmetic durability even when an oil, an oil ingredient that is solid at 25°C, or an organic ultraviolet absorber is added.

Examples of the branched organopolysiloxanes include the following:

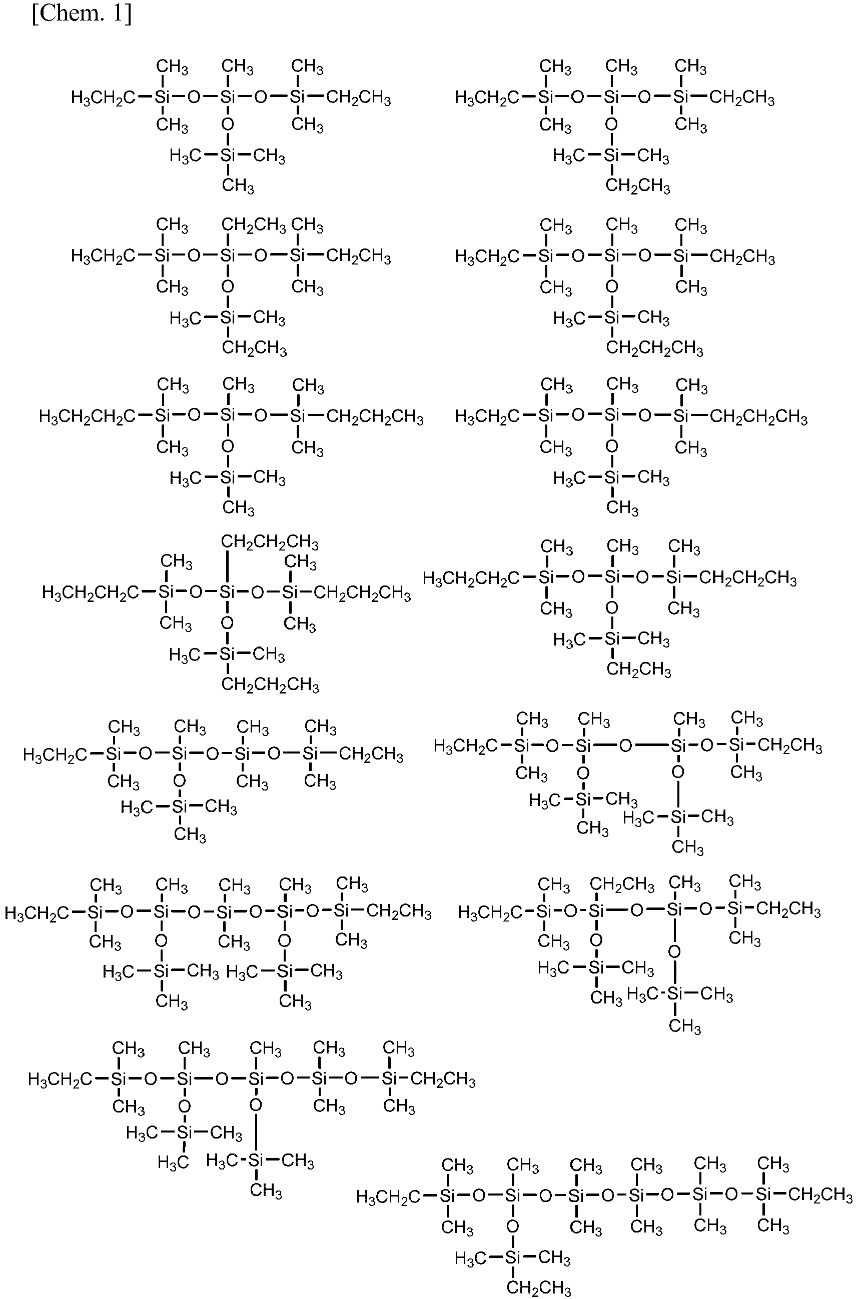

The boiling point of the branched organopolysiloxanes of the present invention is 87 to 119°C at 10 hPa. A cosmetic containing such an organopolysiloxane does not give a dry feeling when applied to the skin, and does not give a strong oily feel after application. If the boiling point is below 87°C, the compound has a high volatilization rate and may give a dry feeling, and further does not allow the cosmetic to spread well easily. If, on the other hand, the boiling point is above 119°C, the cosmetic will have a strong oily feel. The branched organopolysiloxanes in the present invention have a boiling point at 10 hPa in the range of 87 to 119°C and are highly volatile. This allows a cosmetic to offer a good feeling on use after applied to the skin, without producing a strong oily feel. Furthermore, the present invention does not entail strict control of the volatilization rate by, for example, combining a highly volatile organopolysiloxane with a mildly volatile organopolysiloxane. That is, the object described above is achieved even by the use of a single branched organopolysiloxane of the present invention. When two or more of the branched organopolysiloxanes are used in combination, the average of their boiling points should fall in the above range. That is, the above range of the boiling point should be satisfied by one or more of the branched organopolysiloxanes, and a branched organopolysiloxane with a boiling point outside the above range may be added to the cosmetic. In the present invention, the boiling point is the equilibrium reflux boiling point measured by heating the sample at 10 hPa and recording the internal temperature at which the sample is refluxed at a rate of several drops per second.

The branched organopolysiloxane is preferably one or more selected from 1,5-diethyl-3-trimethylsiloxy-1,1,3,5,5-pentamethyltrisiloxane and 1,5-diethyl-3-ethyldimethylsiloxy-1,1,3,5,5-pentamethyltrisiloxane. These branched organopolysiloxanes more reliably allow the cosmetic to offer a good feeling on use without producing a strong oily feel and to attain excellent over-time stability and cosmetic durability even when an oil, an oil ingredient that is solid at 25°C, or an organic ultraviolet absorber is added.

### II. Methods for Producing the Branched Organopolysiloxanes

For example, the branched organopolysiloxane of the present invention may be produced by addition reaction of an organohydrogensiloxane with ethylene or propylene.

Specific examples of the organohydrogensiloxanes include the two types represented by general formulas (2) and (3) below. These organohydrogensiloxanes may be used in combination. 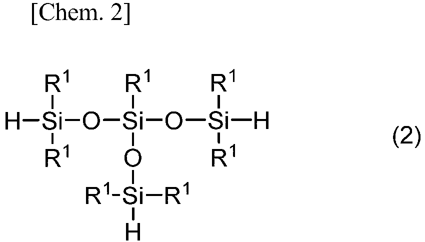
(R¹ is the same as defined above.) 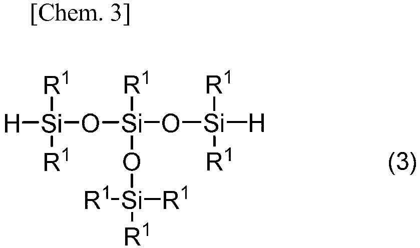
(R¹ is the same as defined above.)

The blending ratio of ethylene or propylene to the organohydrogenpolysiloxane represented by general formula (2) or (3) is preferably 1.0 to 2.0, and more preferably 1.0 to 1.2 in terms of the molar ratio of ethylene or propylene to the hydrosilyl groups.

The addition reaction is desirably performed in the presence of a platinum catalyst or a rhodium catalyst. Specifically, for example, chloroplatinic acid, alcohol-modified chloroplatinic acid, and chloroplatinic acid-vinylsiloxane complex are suitably used. The amount in which the catalyst is used may be a catalytic amount and, in particular, is preferably up to 50 ppm, and more preferably up to 20 ppm in terms of the amount of platinum or rhodium.

The addition reaction may be performed in the absence of a solvent, or an organic solvent may be used as required. Examples of the organic solvents include aromatic hydrocarbons, such as toluene and xylene; aliphatic or alicyclic hydrocarbons, such as n-pentane, n-hexane, and cyclohexane; halogenated hydrocarbons, such as dichloromethane, chloroform, and carbon tetrachloride; ether compounds, such as tetrahydrofuran and dioxane; and ketones, such as acetone and methyl ethyl ketone. The addition reaction is preferably performed in the absence of a solvent or is preferably carried out using a hydrocarbon or an ether compound as an organic solvent.

Although the addition reaction conditions are not particularly limited, it is preferable to carry out the reaction at 40 to 100°C for 1 to 10 hours while introducing ethylene gas or propylene gas into or onto the liquid in the reaction vessel.

### III. Cosmetics

The branched organopolysiloxanes (A) in the present invention have good compatibility with polar oils and the like and are therefore suited for use as solvents and cosmetic oils. The branched organopolysiloxanes in the present invention can be controlled in feeling on use and volatilization rate by combining two or more kinds of the branched organopolysiloxanes having different boiling points. When a slow volatilization rate is desired, a single branched organopolysiloxane may be used in a cosmetic without strict control. When the branched organopolysiloxane (A) of the present invention is added to a cosmetic or the like, the amount thereof is not particularly limited and may be appropriately in the range of 0.01 to less than 100 wt%. The form of the preparation, the purpose of use, and optional ingredients and the amounts thereof are not particularly limited and may be appropriately selected from those that are known. Some optional ingredients are described below. The optional ingredients of each category may be used singly, or two or more may be used in combination.

### (B) Oil ingredients That Are Solid at 25°C

The branched organopolysiloxane (A) in the present invention has high affinity for an oil ingredient that is solid at 25°C and thus may be used together with such an oil ingredient. The branched organopolysiloxane does not inhibit the solidification of the oil ingredient and allows the oil ingredient to be easily manufactured into a stick-shaped preparation or the like that has an expected level of hardness.

The oil ingredient that is solid at 25°C preferably has a melting point of at least 40°C, more preferably 60 to 110°C. Examples include waxes, hydrocarbons, esters, higher alcohols, and higher fatty acids. Any ingredients that are usually added to cosmetics may be used without limitation.

Specific examples include vegetable waxes, such as carnauba wax (INCI: Copernicia Cerifera (Carnauba) Wax), candelilla wax, rice wax, and wood wax; animal waxes, such as beeswax and spermaceti; hydrocarbon waxes, such as solid paraffin, polyethylene, ceresin, ozokerite, and microcrystalline wax; higher alcohols, such as stearyl alcohol, behenyl alcohol, and cetanol; fatty acids, such as stearic acid and behenic acid; silicone waxes, such as Acrylates/Stearyl Acrylate/Dimethicone Methacrylate Copolymer (INCI); and derivatives thereof. One or more selected from those described above is preferably used. Exemplary commercial silicone waxes include KP-561P manufactured by Shin-Etsu Chemical Co., Ltd.

In particular, Polyethylene (INCI), Ceresin (INCI), Ozokerite (INCI), candelilla wax (INCI: Euphorbia Cerifera (Candelilla) Wax), Beeswax (INCI), Microcrystalline Wax (INCI), Stearyl Alcohol (INCI), Behenyl Alcohol (INCI), and cetanol (INCI: Cetyl Alcohol) have high affinity for the branched organopolysiloxanes in the present invention. The branched organopolysiloxanes do not inhibit the solidification of these oil ingredients and allow the oil ingredients to be easily manufactured into a stick-shaped preparation or the like that has an expected level of hardness.

The amount of the oil ingredient that is solid at 25°C is preferably 0.5 to 50 wt% of the cosmetic, and more preferably 2 to 35 wt% of the cosmetic.

### (C) Ultraviolet Absorbers

When ultraviolet shielding effects are desired in the cosmetics of the present invention, it is preferable to add an ultraviolet absorber. The branched organopolysiloxanes in the present invention have excellent compatibility with ultraviolet absorbers. Thus, such cosmetics containing ultraviolet absorbers have a good feeling on use and attain excellent over-time stability and cosmetic durability.

The ultraviolet absorbers are not particularly limited and any ingredients usually added to cosmetics may be used. Specific examples include oxybenzone-1 (INCI: Benzophenone-1), oxybenzone-2 (INCI: Benzophenone-2), oxybenzone-3 (INCI: Benzophenone-3), oxybenzone-4 (INCI: Benzophenone-4), oxybenzone-5 (INCI: Benzophenone-5), oxybenzone-6 (INCI: Benzophenone-6), oxybenzone-9 (INCI: Benzophenone-9), Homosalate (INCI), octocrylene, t-butylmethoxydibenzoylmethane, ethylhexyl salicylate, hexyl diethylaminohydroxybenzoylbenzoate, polysilicone-15, octyl dimethoxybenzylidenedioxoimidazolidinepropionate (INCI: Ethylhexyl Dimethoxybenzylidene Dioxoimidazolidine Propionate), Terephthalylidene Dicamphor Sulfonic Acid (INCI), Ethylhexyltriazone (INCI), methylbis(trimethylsiloxy)silylisopentyl trimethoxycinnamate (INCI: Isopentyl Trimethoxycinnamate Trisiloxane), Drometrizole Trisiloxane (INCI), Ethylhexyl Dimethyl PABA (INCI), Isopropyl Methoxycinnamate (INCI), ethylhexyl methoxycinnamate, bisethylhexyloxyphenol methoxyphenyl triazine, Phenylbenzimidazole Sulfonic Acid (INCI), methylenebisbenzotriazolyltetramethylbutylphenol, Glyceryl Ethylhexanoate Dimethoxycinnamate (INCI), Glyceryl PABA (INCI), Diisopropyl Methyl Cinnamate (INCI), Cinoxate (INCI), and Ethylhexyl Dimethoxybenzylidene Dioxoimidazolidine Propionate (INCI).

Furthermore, a UVA absorber (such as, for example, hexyl diethylaminohydroxybenzoylbenzoate) and a UVB absorber (such as, for example, ethylhexyl methoxycinnamate) may be used in combination, or absorbers belonging to the same type may be combined appropriately.

In this case, it is preferable that the ultraviolet absorber be one or more organic ultraviolet absorbers selected from Ethylhexyl Methoxycinnamate (INCI), hexyl diethylaminohydroxybenzoylbenzoate (INCI: Diethylamino Hydroxybenzoyl Hexyl Benzoate), Ethylhexyl Salicylate (INCI), Polysilicone-15 (INCI), t-butylmethoxydibenzoylmethane (INCI: Butyl Methoxydibenzoylmethane), oxybenzone (INCI: Benzophenone), Methylenebisbenzotriazolyltetramethylbutylphenol (INCI), Bisethylhexyloxyphenolmethoxyphenyltriazine (INCI), and Octocrylene (INCI). These organic ultraviolet absorbers, in particular, advantageously offer higher ultraviolet absorbing effects and exhibit excellent compatibility with the branched organopolysiloxanes of the present invention.

The amount of the ultraviolet absorber is preferably 1 to 50 wt% of the cosmetic, and more preferably 3 to 20 wt% of the cosmetic.

### <Other Optional Ingredients>

The cosmetic may contain other optional ingredients, for example, (1) oils other than (B), (2) compounds having an alcoholic hydroxyl group, (3) surfactants, (4) powders, (5) compositions including a crosslinked organopolysiloxane and an oil that is liquid at room temperature, (6) film-forming agents, (7) antiperspirants, (8) antimicrobial agents, and (9) other additives. These may be used singly in an appropriate amount, or two or more may be appropriately combined in appropriate amounts.

### (1) Oils Other Than (B)

The optional oils may be semi-solid or liquid oils other than the oil ingredients that are solid at 25°C. Examples include natural animal and vegetable oils and fats, semi-synthetic oils and fats, hydrocarbon oils, higher fatty acids, higher alcohols, esters, silicone oils other than the branched organopolysiloxanes that are the essential ingredients for achieving the advantageous effects of the present invention, and fluorine-based oils.

### • Natural Animal and Vegetable Oils and Fats, and Semi-Synthetic Oils and Fats

Examples of the natural animal and vegetable oils and the semi-synthetic oils include natural vegetable oils, such as avocado oil (INCI: Persea Gratissima (Avocado) Oil), linseed oil (INCI: Linum Usitatissimum (Linseed) Seed Oil), almond oil (INCI: Prunus Amygdalus Dulcis (Sweet Almond) Oil), perilla oil, olive oil (INCI: Olea Europaea (Olive) Fruit Oil), Torreya californica oil (INCI: Torreya Californica (California Nutmeg) Oil), citronella grass oil (INCI: Cymbopogon Nardus (Citronella) Oil), Torreya Nucifera Seed Oil (INCI), apricot kernel oil (INCI: Kyounin Yu), wheat germ oil (INCI: Triticum Vulgare (Wheat) Germ Oil), sesame oil (INCI: Sesamum Indicum (Sesame) Seed Oil), wheat germ oil (INCI: Triticum Vulgare (Wheat) Germ Oil), rice germ oil (INCI: Oryza Sativa (Rice) Germ Oil), rice bran oil (INCI: Oryza Sativa (Rice) Bran Oil), sasanqua oil (INCI: Camellia Kissi Seed Oil), safflower oil (INCI: Carthamus Tinctorius (Safflower) Seed Oil), soybean oil (INCI: Glycine Soja (Soybean) Oil), tea seed oil (INCI: Camellia Sinensis Seed Oil), camellia oil (INCI: Camellia Japonica Seed Oil), evening primrose oil (INCI: Oenothera Biennis (Evening Primrose) Oil), rapeseed oil (INCI: RAPE SHUSHI YU), germ oils, for example, corn germ oil (INCI: Zea Mays (Corn) Germ Oil) and wheat germ oil (INCI: Triticum Vulgare (Wheat) Germ Oil), persic oil, palm oil (INCI: Elaeis Guineensis (Palm) Oil), palm kernel oil (INCI: Elaeis Guineensis (Palm) Kernel Oil), castor oil (INCI: Ricinus Communis (Castor) Seed Oil), sunflower oil (INCI: Helianthus Annuus (Sunflower) Seed Oil), grape seed oil (INCI: Vitis Vinifera (Grape) Seed Oil), Jojoba seed oil (INCI: Simmondsia Chinensis (Jojoba) Seed Oil), macadamia seed oil (INCI: Macadamia Ternifolia Seed Oil), meadowfoam oil (INCI: Limnanthes Alba (Meadowfoam) Seed Oil), cotton seed oil (INCI: Gossypium Herbaceum (Cotton) Seed Oil), coconut oil (INCI: Cocos Nucifera (Coconut) Oil), and peanut oil (INCI: Arachis Hypogaea (Peanut) Oil); natural animal oils, such as Shark Liver Oil (INCI), Cod Liver Oil (INCI), Fish Liver Oil (INCI), Turtle Oil (INCI), Mink Oil (INCI), and egg yolk oil (INCI: Egg Oil); and semi-synthetic oils and fats, such as Hydrogenated Coconut Oil (INCI) and liquid lanolin (INCI: Lanolin Oil).

### • Hydrocarbon Oils

Examples of the hydrocarbon oils include linear or branched hydrocarbon oils. The hydrocarbon oils may be volatile or non-volatile.

Specific examples include alkanes, such as Olefin Oligomers (INCI), isoparaffins, for example, (C13, 14) Isoparaffins (INCI), Isododecane (INCI), Undecane (INCI), Dodecane (INCI), Isohexadecane (INCI), Hydrogenated Polyisobutene (INCI), Squalane (INCI), Mineral Oil (INCI), Coconut Alkanes (INCI), (C13-15) Alkanes (INCI), and vaseline (INCI: Petrolatum).

### • Higher Fatty Acids

Examples of the higher fatty acids include Oleic Acid (INCI), Linoleic Acid (INCI), Linolenic Acid (INCI), Arachidonic Acid (INCI), Eicosapentaenoic Acid (INCI), Docosahexaenoic Acid (INCI), Isostearic Acid (INCI), and Hydroxystearic Acid (INCI).

### • Higher Alcohols

For example, the higher alcohols are preferably C6 and higher alcohols. Specific examples of the higher alcohols include Lauryl Alcohol (INCI), Myristyl Alcohol (INCI), Palmityl Alcohol (INCI), Stearyl Alcohol (INCI), Behenyl Alcohol (INCI), Oleyl Alcohol (INCI), Isostearyl Alcohol (INCI), Octyldodecanol (INCI), Cholesterol (INCI), Phytosterols (INCI), and Batyl Alcohol (INCI).

### • Ester Oils

Examples of the ester oils include Diisobutyl Adipate (INCI), dihexyldecyl adipate, Diheptylundecyl Adipate (INCI), n-alkyl glycol monoisostearates, such as Isostearyl Isostearate (INCI), Isocetyl Isostearate (INCI), Trimethylolpropane Triisostearate (INCI), Glycol Diethylhexanoate (INCI), Cetyl Ethylhexanoate (INCI), Trimethylolpropane Triethylhexanoate (INCI), Pentaerythrityl Tetraethylhexanoate (INCI), cetyl octanoate (INCI: Cetyl Ethylhexanoate), octyldodecyl esters, such as octyldodecyl stearoyloxystearate (INCI: Octyldodecyl Stearoyl Stearate), Oleyl Oleate (INCI), Octyldodecyl Oleate (INCI), Decyl Oleate (INCI), neopentyl glycol dioctanoate (INCI: Neopentyl Glycol Diethylhexanoate), Neopentyl Glycol Dicaprate (INCI), Diisostearyl Malate (INCI), Triethyl Citrate (INCI), Diethylhexyl Succinate (INCI), Amyl Acetate (INCI), Ethyl Acetate (INCI), Butyl Acetate (INCI), Isocetyl Stearate (INCI), Butyl Stearate (INCI), Diisopropyl Sebacate (INCI), Diethylhexyl Sebacate (INCI), Cetyl Lactate (INCI), Myristyl Lactate (INCI), Isononyl Isononanoate (INCI), Isotridecyl Isononanoate (INCI), palmitic acid esters, such as Isopropyl Palmitate (INCI), ethylhexyl palmitate (INCI: Ethylhexyl Isopalmitate), and hexyldecyl palmitate (INCI: Isocetyl Palmitate, Hexyldecyl Palmitate), Cholesteryl Hydroxystearate (INCI), myristic acid esters, such as Isopropyl Myristate (INCI), Octyldodecyl Myristate (INCI), and Myristyl Myristate (INCI), Ethylhexyl Laurate (INCI), Hexyl Laurate (INCI), Dioctyldodecyl Lauroyl Glutamate (INCI), and Isopropyl Lauroyl Sarcosinate (INCI).

Among the ester oils, glyceride oils include Triethylhexanoin (INCI), glyceryl tri(caprylate/caprate) (INCI: Caprylic/Capric Triglyceride), Cocoglyceride (INCI), triglyceryl (caprylate/caprate/succinate) (INCI: Caprylic/Capric/Succinic Triglyceride), and Caprylic/Capric Glycerides (INCI).

### • Silicone Oils

The silicone oils may be silicone oils other than the branched organopolysiloxanes that are the essential ingredients for achieving the advantageous effects of the present invention.

Examples include alkyl-modified silicones, such as Dimethicone (INCI), Trisiloxane (INCI), Methyltrimethicone (INCI), Ethyltrisiloxane (INCI), Ethylmethicone (INCI), and Hexyldimethicone (INCI), long-chain alkyl-modified silicones, such as Caprylylmethicone (INCI), low-viscosity to high-viscosity, linear or branched organopolysiloxanes, such as Phenyl Trimethicone (INCI), Diphenyl Dimethicone (INCI), Diphenylsiloxyphenyl Trimethicone (INCI), Tetraphenyldimethyldisiloxane (INCI), and methylhydrogenpolysiloxane, cyclic organopolysiloxanes, such as Cyclotetrasiloxane (INCI), Cyclopentasiloxane (INCI), and Cyclohexasiloxane (INCI), amino-modified organopolysiloxanes, such as Amodimethicone (INCI) and Aminopropyl Dimethicone (INCI), pyrrolidone-modified organopolysiloxanes, such as PCA Dimethicone (INCI), pyrrolidonecarboxylic acid-modified organopolysiloxanes, silicone rubbers, such as high degree of polymerization gum-like dimethylpolysiloxanes, gum-like amino-modified organopolysiloxanes, and gum-like dimethylsiloxane-methylphenylsiloxane copolymers, low-viscosity organopolysiloxane solutions of a silicone gum or rubber, amino acid-modified silicones, fluorine-modified silicones, silicone resins, and dissolved silicone resins.

Examples of the commercial silicone oils include KF-96L-1cs, KF-96L-1.5cs, KF-96L-2cs, KF-96A-6cs, KF-4422, KF-54, KF-54HV, KF-56A, and KF-995 manufactured by Shin-Etsu Chemical Co., Ltd.

### • Fluorine-Based Oils

Examples of the fluorine-based oils include Perfluorodecalin (INCI), Perfluorononyl Dimethicone (INCI), and Perfluoromethylcyclopentane (INCI).

### (2) Compounds Having an Alcoholic Hydroxyl Group

Examples of the compounds having an alcoholic hydroxyl group include lower alcohols, preferably those having 2 to 5 carbon atoms, such as ethanol (INCI: Alcohol) and isopropanol (INCI: Isopropyl Alcohol), and sugar alcohols, such as Sorbitol (INCI), Maltose (INCI), and Xylitol (INCI). Examples further include sterols, such as Cholesterol (INCI), sitosterol (INCI: Beta-Sitosterol), Phytosterols (INCI), and Lanosterol (INCI).

### (3) Surfactants

The surfactants may be nonionic, anionic, cationic, or amphoteric and any surfactants usually added to cosmetics may be used without limitation. In particular, preferred surfactants are partially crosslinked polyether-modified silicones, partially crosslinked polyglycerin-modified silicones, linear or branched polyoxyethylene-modified organopolysiloxanes, linear or branched polyoxyethylene polyoxypropylene-modified organopolysiloxanes, linear or branched polyoxyethylene/alkyl co-modified organopolysiloxanes, linear or branched polyoxyethylene polyoxypropylene/alkyl co-modified organopolysiloxanes, linear or branched polyglycerin-modified organopolysiloxanes, and linear or branched polyglycerin/alkyl co-modified organopolysiloxanes. In these surfactants, the content of hydrophilic polyoxyethylene groups, polyoxyethylene polyoxypropylene groups, or polyglycerin residues is preferably 10 to 70 wt% of the molecule.

Examples of the partially crosslinked polyether-modified silicones and the partially crosslinked polyglycerin-modified silicones include (Dimethicone/(PEG-10/15) Crosspolymer (INCI), (PEG-15/Lauryl Dimethicone) Crosspolymer (INCI) , (PEG-10/Lauryl Dimethicone) Crosspolymer (INCI), (PEG-15/Lauryl Polydimethylsiloxyethyl Dimethicone) Crosspolymer (INCI), (Dimethicone/Polyglycerin-3) Crosspolymer (INCI), (Lauryl Dimethicone/Polyglycerin-3) Crosspolymer (INCI), and (Polyglycerin-3/Lauryl Polydimethylsiloxyethyl Dimethicone) Crosspolymer (INCI).

When the partially crosslinked polyether-modified silicone or the partially crosslinked polyglycerin-modified silicone is used, the crosslinked organopolysiloxane preferably forms a composition with an oil that is liquid at room temperature in such a manner that the crosslinked organopolysiloxane is swollen with its own weight or more of the liquid oil.

The liquid oil may be the branched organopolysiloxane in the present invention or may be the optional ingredient (1), specifically, a liquid silicone oil, a hydrocarbon oil, an ester oil, a natural animal or vegetable oil, a semi-synthetic oil, or a fluorine-based oil. Examples include Cyclopentasiloxane (INCI), Dimethicone (INCI), Mineral Oil (INCI), Isododecane (INCI), Isohexadecane (INCI), Triethylhexanoin (INCI), Isotridecyl Isononanoate (INCI), and Squalane (INCI).

Examples of the commercial crosslinked organopolysiloxanes swollen with liquid oils include KSG-210, KSG-240, KSG-270, KSG-310, KSG-320, KSG-330, KSG-340, KSG-320Z, KSG-350Z, KSG-710, KSG-810, KSG-820, KSG-830, KSG-840, KSG-820Z, and KSG-850Z manufactured by Shin-Etsu Chemical Co., Ltd.

Exemplary surfactants that are not crosslinked organopolysiloxanes include PEG-11 Methyl Ether Dimethicone (INCI), PEG/PPG-20/22 Butyl Ether Dimethicone (INCI), PEG-3 Dimethicone (INCI), PEG-10 Dimethicone (INCI), PEG-9 Polydimethylsiloxyethyl Dimethicone (INCI), Lauryl PEG-9 Polydimethylsiloxyethyl Dimethicone (INCI), Cetyl PEG/PPG-10/1 Dimethicone (INCI), Polyglyceryl-3 Disiloxane Dimethicone (INCI), Polyglyceryl-3 Polydimethylsiloxyethyl Dimethicone (INCI), and Lauryl Polyglyceryl-3 Polydimethylsiloxyethyl Dimethicone (INCI).

Exemplary commercial products include KF-6011, KF-6011P, KF-6012, KF-6015, KF-6017, KF-6043, KF-6028, KF-6038, KF-6048, KF-6100, KF-6104, KF-6106, KF-6105, and KF-6115 manufactured by Shin-Etsu Chemical Co., Ltd.

For any types of surfactants, the amount is preferably 0.1 to 20 wt% of the whole of the cosmetic. When the amount is at least 0.1wt%, the surfactant can fully fulfill its dispersing and emulsifying functions. When the amount is 20 wt% or less, the cosmetic will not give a sticky feeling on use and is therefore preferable. The HLB of the surfactant is not limited but is preferably 2 to 14.5 in order to maintain the water resistance of the cosmetic.

### (4) Powders

Examples of the powders include coloring pigments, inorganic powders, metal powders, organic powders, and inorganic/organic composite powders. The powders will be specifically described below.

### • Coloring Pigments

The coloring pigments are not particularly limited and any pigments that are usually added to give colors to cosmetics may be used. Examples include red iron oxide (INCI: Iron Oxides), yellow iron oxide (INCI: Iron Oxides), white titanium oxide (INCI: Titanium Dioxide), black iron oxide (INCI: Iron Oxides), Ultramarines (INCI), iron blue (INCI: Ferric Ferrocyanide, Ferric Ammonium Ferrocyanide), Manganese Violet (INCI), Cobalt Titanium Oxide (INCI), chromium hydroxide (INCI: Chromium Hydroxide Green), chromium oxide (INCI: Chromium Oxide Greens), Cobalt Aluminum Oxide (INCI), Cobalt Titanium Oxide (INCI), fired (titanium/titanium oxide) (INCI: Titanium/Titanium Dioxide), Lithium Cobalt Titanate (INCI), Cobalt Titanium Oxide (INCI), sintered (iron oxide/titanium oxide), composites doped with dissimilar metals, such as iron oxide doped titanium oxide (INCI: Iron Oxides, Titanium Dioxide), Titanium Nitride (INCI), ferrous hydroxide (INCI: Iron Hydroxide), inorganic brown pigments, such as γ-iron oxide, inorganic yellow pigments, such as loess, and colored pigments, such as lakes of tar dyes and lakes of natural dyes.

The pigments may have any shapes, such as spherical, approximately spherical, rod-like, spindle-like, petal-like, strip-like, and amorphous shapes. The geometric forms of the pigments are not particularly limited as long as the pigments can impart colors to the cosmetics.

### • Inorganic Powders

Examples of the inorganic powders include fine particles of zirconium oxide (INCI: Zirconium Dioxide), Zinc Oxide (INCI), Cerium Oxide (INCI), Magnesium Oxide (INCI), Barium Sulfate (INCI), calcium sulfate (INCI: Calcium Sulfate), Magnesium Sulfate (INCI), Calcium Carbonate (INCI), Magnesium Carbonate (INCI), Talc (INCI), Mica (INCI), Kaolin (INCI), Synthetic Fluorphlogopite (INCI), synthetic ferrous phlogopite, black mica (INCI: Biotite), Potassium Silicate (INCI), Silica (INCI), Aluminum Silicate (INCI), Magnesium Silicate (INCI), Magnesium Aluminum Silicate (INCI), Calcium Silicate (INCI), Aluminum Calcium Sodium Silicate (INCI), Lithium Magnesium Sodium Silicate (INCI), Sodium Magnesium Silicate (INCI), Calcium Aluminum Borosilicate (INCI), Calcium Sodium Borosilicate (INCI), Hydroxyapatite (INCI), Bentonite (INCI), Montmorillonite (INCI), Hectorite (INCI), Zeolite (INCI), Alumina (INCI), Aluminum Hydroxide (INCI), Boron Nitride (INCI), and Glass (INCI).

Inorganic coloring pearl pigments include pearl agents, such as Mica (INCI) coated with titanium oxide (INCI: Titanium Dioxide) and Synthetic Fluorophlogopite (INCI) coated with titanium oxide (INCI: Titanium Dioxide), and pearl pigments, such as Bismuth Oxychloride (INCI), Bismuth Oxychloride (INCI) coated with titanium oxide (INCI: Titanium Dioxide), Talc (INCI) coated with titanium oxide (INCI: Titanium Dioxide), fish scale flakes, and coloring mica coated with titanium oxide (INCI: Titanium Dioxide). These pigments may be surface-treated by a known method generally used for cosmetics or may be free from such surface treatment.

### • Metal Powders

Examples of the metal powders include metal fine particles of aluminum (INCI: Aluminum, Aluminum Powder), copper (INCI: Copper Powder), silver (INCI: Silver Powder), and Gold (INCI).

### • Organic Powders

Examples of the organic powders include powders of silicone, polyamide, polyacrylic acid/acrylic acid ester, polyester, Polyethylene (INCI), Polypropylene (INCI), Polystyrene (INCI), styrene/acrylic acid copolymer, divinylbenzene/styrene copolymer, polyurethane, vinyl resin, urea resin, melamine resin, benzoguanamine, polymethylbenzoguanamine, tetrafluoroethylene, polymethyl methacrylate, Cellulose (INCI), Silk (INCI), nylon, phenolic resin, epoxy resin, and polycarbonate.

In particular, examples of the silicones include silicone resin particles; Polymethylsilsesquioxane (INCI), silicone rubber powder, silicone resin-coated silicone rubber powder; (Vinyl Dimethicone/Methicone Silsesquioxane) Crosspolymer (INCI), (Diphenyl Dimethicone/Vinyldiphenyl Dimethicone/Silsesquioxane) Crosspolymer (INCI), Polysilicone-1 Crosspolymer (INCI), and Polysilicone-22 (INCI).

Examples of the commercial silicone powders include KMP-590, KMP-591, KMP-592, KMP-597, KMP-598, KSP-100, KSP-101, KSP-102, KSP-105, KSP-300, KSP-411, KSP-441, KM-9729, and KM-440 manufactured by Shin-Etsu Chemical Co., Ltd.

Examples further include metal soaps. Specific examples include powders of Zinc Stearate (INCI), Aluminum Stearate (INCI), Calcium Stearate (INCI), Magnesium Stearate (INCI), Zinc Myristate (INCI), Magnesium Myristate (INCI), Sodium Zinc Cetyl Phosphate (INCI), and Potassium Cetyl Phosphate (INCI).

Examples further include organic dyes. Specific examples include tar dyes, such as red 3, red 104 (1) (INCI: Red 28, Red 28 Lake), red 106, red 201 (INCI: Red 6), red 202 (INCI: Red 7), red 204, red 205, red 220 (INCI: Red 34), red 226 (INCI: Red 30), red 227 (INCI: Red 33, RED 33 Lake), red 228 (INCI: Red 36), red 230 (1) (INCI: Red 22, Red 22 Lake), red 230 (2), red 401, red 505, yellow 4 (INCI: Yellow 5), yellow 5 (INCI: Yellow 6, Yellow 6 Lake), yellow 202 (1) (INCI: Yellow 8), yellow 203 (INCI: Yellow 10, Yellow 10 Lake), yellow 204 (INCI: Yellow 11), yellow 401, blue 1 (INCI: Blue 1, Blue 1 Lake), blue 2, blue 201, blue 205 (INCI: Blue 4), blue 404, green 3 (INCI: Green 3, Green 3 Lake), green 201 (INCI: Green 5), green 202 (INCI: Green 6), green 204 (INCI: Green 8), green 205, orange 201 (INCI: Orange 5), orange 203 (INCI: Pigment Orange 5), orange 204, orange 205 (INCI: Orange 4, Orange 4 Lake), orange 206 (INCI: Orange 10), and orange 207 (INCI: Orange 11), and natural dyes, such as Cochineal (INCI), Laccaic Acid (INCI), safflower red (INCI: Carthamus Tinctorius (Safflower) Flower Extract), Lithospermum Officinale Root Extract (INCI), gardenia yellow, and gardenia blue (INCI: Hydrolyzed Gardenia Florida Extract).

### • Inorganic/Organic Composite Powders

Examples of the inorganic/organic composite powders include composite powders in which the surface of an inorganic powder is coated with an organic powder by a publicly known method.

The powders described hereinabove may be surface-treated. From the point of view of the water resistance of the cosmetic, the surface treatment agent is preferably one that can impart hydrophobicity. The hydrophobicity-imparting agent is not particularly limited, and examples thereof include silicone treatment agents, waxes, paraffins, organic fluorine compounds, such as perfluoroalkyls and phosphate salts, surfactants, amino acids, such as N-acylglutamic acid, and metal soaps, such as aluminum stearate and magnesium myristate.

Silicone treatment agents are more preferable. Examples thereof include silanes or silylating agents, such as Triethoxycaprylylsilane (INCI), Dimethicone (INCI), Methicone (INCI), Hydrogen Dimethicone (INCI), Triethoxysilylethyl Polydimethylsiloxyethyl Dimethicone (INCI), Triethoxysilylethyl Polydimethylsiloxyethylhexyl Dimethicone (INCI), and Acrylates/Tridecyl Acrylate/Triethoxysilylpropyl Methacrylate/Dimethicone Methacrylate Copolymer (INCI). Specific examples of the silicone treatment agents include AES-3083, KF-99P, KF-9901, KF-9908, KF-9909, KP-574, and KP-541 manufactured by Shin-Etsu Chemical Co., Ltd.

The surface hydrophobizing agents may be used singly, or two or more may be used in combination. Specific examples of the surface-treated coloring pigments include KTP-09 series, in particular, KTP-09W, 09R, 09Y, and 09B manufactured by Shin-Etsu Chemical Co., Ltd.

### (5) Compositions Including a Crosslinked Organopolysiloxane and an Oil That is Liquid at Room Temperature

In the composition including a crosslinked organopolysiloxane and an oil that is liquid at room temperature, the crosslinked organopolysiloxane is preferably swollen by containing its own weight or more of the liquid oil. The liquid oil may be the branched organopolysiloxane in the present invention or may be the optional ingredient (1), specifically, a liquid silicone oil, a hydrocarbon oil, an ester oil, a natural animal or vegetable oil, a semi-synthetic oil, or a fluorine-based oil. Examples include Cyclopentasiloxane (INCI), Dimethicone (INCI), Mineral Oil (INCI), Isododecane (INCI), Isohexadecane (INCI), Triethylhexanoin (INCI), Isotridecyl Isononanoate (INCI), and Squalane (INCI).

Unlike the ingredients (3), the ingredients (5) are compounds that do not have a polyether or polyglycerin structure in the molecular structure. Specific examples include (Dimethicone/Vinyl Dimethicone) Crosspolymer (INCI), (Dimethicone/Phenylvinyl Dimethicone) Crosspolymer (INCI), (Vinyl Dimethicone/Lauryl Dimethicone) Crosspolymer (INCI), and (Lauryl Polydimethylsiloxyethyl Dimethicone/Bisvinyl Dimethicone) Crosspolymer (INCI).

Examples of the commercial compositions including a crosslinked organopolysiloxane and an oil that is liquid at room temperature include KSG-15, KSG-1510, KSG-16, KSG-1610, KSG-19, KSG-016F, KSG-18A, KSG-41A, KSG-42A, KSG-43, KSG-44, KSG-042Z, KSG-045Z, and KSG-048Z manufactured by Shin-Etsu Chemical Co., Ltd.

### (6) Film-Forming Agents

The film-forming agent is added mainly for the purpose of further increasing the durability of the effects of the cosmetic. The film-forming agents are not particularly limited but are preferably silicone compositions from the point of view of imparting water repellency. Specifically, for example, trimethylsiloxysilicate, acrylic-silicone coating agents, silicone-modified norbornenes, and silicone-modified pullulans may be used.

Examples of the silicone compositions as the film-forming agents include Trimethylsiloxysilicate (INCI), (Acrylates/Dimethicone) Copolymer (INCI), (Norbomene/Tris(trimethylsiloxy)silylnorbomene) Copolymer (INCI), and pullulan tri(trimethylsiloxy)silylpropylcarbamate (INCI: Trimethylsiloxysilylcarbamoyl Pullulan).

The film-forming agent may be added to the cosmetic after being previously dissolved into an oil that is liquid at room temperature. The liquid oil may be the branched organopolysiloxane in the present invention or may be the optional ingredient (1), specifically, a liquid silicone oil, a hydrocarbon oil, an ester oil, a natural animal or vegetable oil, a semi-synthetic oil, or a fluorine-based oil. Examples include Cyclopentasiloxane (INCI), Dimethicone (INCI), Mineral Oil (INCI), Isododecane (INCI), Isohexadecane (INCI), Triethylhexanoin (INCI), Isotridecyl Isononanoate (INCI), Squalane (INCI), and Butyl Acetate (INCI). Specific examples of the commercial silicone film-forming agents include KF-7312J, KP-545, KP-549, KP-543, NBN-30-ID, TSPL-30-ID, and TSPL-30-D5 manufactured by Shin-Etsu Chemical Co., Ltd.

### (7) Antiperspirants

When the cosmetic according to the present invention is a deodorant, an antiperspirant may be optionally added. The antiperspirant is not particularly limited as long as it shrinks the skin pores to suppress perspiration. General such ingredients may be widely used. Examples include halogenated hydroxyaluminums, such as chlorohydroxyaluminum and allantoin chlorohydroxyaluminum, aluminum halides, such as aluminum chloride, allantoin aluminum salt, tannic acid, persimmon tannin, aluminum potassium sulfate, zinc oxide, zinc p-phenolsulfonate, burnt alum, tetrachloro(Al/zirconium) hydrate, and trichlorohydrex glycine (Al/zirconium). In order to obtain high effects, in particular, the antiperspirant active ingredient is preferably selected from the group consisting of aluminum halides, halogenated hydroxyaluminums, and complexes or mixtures thereof with zirconyl oxyhalides and zirconyl hydroxyhalides.

The antiperspirant may be used as a solution in water, or a powder thereof may be added directly to the preparation. Commercially available antiperspirants may be used. The commercially available product may be in the form of a mixed ingredient containing other ingredients. The content of the antiperspirant is not particularly limited and may be changed appropriately depending on the amounts of other ingredients that are blended. In order to obtain a deodorant with excellent antiperspirant effects and to reduce the skin irritation of the deodorant, the content is preferably in the range of 0.001 to 30 wt%, and more preferably in the range of 0.01 to 20 wt% of the whole of the deodorant.

### (8) Antimicrobial Agents

The antimicrobial agents are not particularly limited as long as the ingredients can provide deodorizing effects by suppressing the proliferation of bacteria resident on the skin that produce odor-causing substances. For example, such antimicrobial agents as triclosan, benzalkonium chloride, benzethonium chloride, chlorhexidine hydrochloride, chlorhexidine gluconate, halocarban, and isomethylphenol are generally used. Furthermore, for example, crude drug-derived essential oils or extracts having antibacterial properties, such as green tea dry distillation extract, may be added. Examples of the antimicrobial agents that are crude drug-derived essential oils or extracts having deodorizing effects include green tea extract, lavender extract, Scutellaria baicalensis Georgi extract, Coptis japonica extract, Phellodendron Amurense Bark extract, Artemisia capillaris extract, Aloe arborescens extract, Sophora flavescens root extract, Sasa veitchii leaf extract, Allium sativum extract, Hamamelis extract, black tea extract, Salvia officinalis leaf extract, Zanthoxylum fruit extract, Zingiber Officinale root extract, Acorus Calamus root extract, Hedera Helix extract, Houttuynia Cordata extract, Prunus Persica fruit extract, Prunus Persica leaf extract, Mentha Piperita leaf extract, Cnidium Officinale root extract, Eucalyptus Globulus leaf extract, Arachis Hypogaea seedcoat extract, Ganoderma Lucidum extract, and Sanguisorba Officinalis root extract. The plant extracts may be used singly, or two or more may be used in combination.

### (9) Other Additives

Other additives include oil-soluble gelling agents, ultraviolet absorbing and scattering agents, moisturizers, preservatives, fragrances, salts, antioxidants, pH adjusters, chelating agents, algefacients, anti-inflammatory agents, skin beautifying ingredients (such as whitening agents, cell activators, skin roughness improvers, circulation promoting ingredients, skin astringents, and antiseborrheic agents), vitamins, amino acids, nucleic acids, hormones, and clathrate compounds.

### • Oil-Soluble Gelling Agents

Examples of the oil-soluble gelling agents include metal soaps, such as aluminum stearate, magnesium stearate, and zinc myristate; amino acid derivatives, such as Lauroyl Glutamic Acid (INCI) and α,γ-di-n-butylamine; dextrin fatty acid esters, such as Dextrin Palmitate (INCI), Dextrin Isostearate (INCI), Dextrin Myristate (INCI), inulin stearate (INCI: Stearoyl Inulin), and Dextrin Palmitate/Ethylhexanoate (INCI); sucrose fatty acid esters, such as sucrose palmitate and sucrose stearate; fructooligosaccharide fatty acid esters, such as fructooligosaccharide stearate and fructooligosaccharide 2-ethylhexanoate; benzylidene derivatives of sorbitol, such as monobenzylidene sorbitol and dibenzylidene sorbitol; organically modified clay minerals of hectorite, such as Disteardimonium Hectorite (INCI) and Stearalkonium Hectorite (INCI); and Stearalkonium Bentonite (INCI).

### • Ultraviolet Absorbing and Scattering Agents

Examples of the ultraviolet absorbing and scattering agents include particles that absorb and scatter ultraviolet rays, such as fine titanium oxide particles, fine iron-containing titanium oxide particles, fine zinc oxide particles, fine cerium oxide particles, and composites thereof. These ultraviolet absorbing and scattering particles may be dispersed in an oil beforehand. The oil may be the branched organopolysiloxane in the present invention or may be the optional ingredient (1), specifically, a liquid silicone oil, a hydrocarbon oil, an ester oil, a natural animal or vegetable oil, a semi-synthetic oil, or a fluorine-based oil. Examples include Cyclopentasiloxane (INCI), Dimethicone (INCI), Mineral Oil (INCI), Isododecane (INCI), Isohexadecane (INCI), Triethylhexanoin (INCI), Isotridecyl Isononanoate (INCI), and Squalane (INCI).

Specific examples of the oil dispersions of the ultraviolet absorbing and scattering particles include SPD series (product name), in particular, SPD-T5, Z5, T6, Z6, and T7 manufactured by Shin-Etsu Chemical Co., Ltd.

### • Moisturizers

Examples of the moisturizers include polyhydric alcohols, such as BG (INCI: Butylene Glycol), PG (INCI: Propylene Glycol), DPG (INCI: Dipropylene Glycol), Pentylene Glycol (INCI), 1,10-Decanediol (INCI), Octanediol (INCI), 1,2-Hexanediol (INCI), Erythritol (INCI), Glycerin (INCI), Diglycerin (INCI), and polyethylene glycol; Glucose (INCI), Glyceryl Glucoside (INCI), Betaine (INCI), Sodium Chondroitin Sulfate (INCI), Sodium PCA (INCI), Methyl Gluceth-10 (INCI), Methyl Gluceth-20 (INCI), hyaluronic acid, egg yolk lecithin, soybean lecithin, phosphatidylcholine, phosphatidylethanolamine, phosphatidylserine, phosphatidylglycerol, phosphatidylinositol, and sphingophospholipid.

### • Preservatives

Examples of the preservatives include p-oxybenzoic acid alkyl esters, benzoic acid, sodium benzoate, sorbic acid, potassium sorbate, and phenoxyethanol. Examples of the antimicrobial agents include benzoic acid, salicylic acid, carbolic acid, sorbic acid, p-oxybenzoic acid alkyl esters, p-chloro-m-cresol, hexachlorophene, trichlorocarbanilide, photosensitizers, and phenoxyethanol.

### • Fragrances

The fragrances include natural fragrances and synthetic fragrances. Examples of the natural fragrances include plant fragrances separated from, for example, flowers, leaves, wood, and pericarps; and animal fragrances, such as musk and civet. Examples of the synthetic fragrances include hydrocarbons, such as monoterpenes; alcohols, such as aliphatic alcohols and aromatic alcohols; aldehydes, such as terpene aldehydes and aromatic aldehydes; ketones, such as alicyclic ketones; esters, such as terpene esters; lactones; phenols; oxides; nitrogen-containing compounds; and acetals.

### • Salts

The salts include inorganic salts, organic acid salts, amine salts, and amino acid salts. Examples of the inorganic salts include sodium salts, potassium salts, magnesium salts, calcium salts, aluminum salts, zirconium salts, and zinc salts of inorganic acids, such as hydrochloric acid, sulfuric acid, carbonic acid, and nitric acid. Examples of the organic acid salts include salts of organic acids, such as acetic acid, dehydroacetic acid, citric acid, malic acid, succinic acid, ascorbic acid, and stearic acid. Examples of the amine salts and the amino acid salts include salts of amines, such as triethanolamine, and salts of amino acids, such as glutamic acid. Furthermore, use may be made of, for example, salts of hyaluronic acid and chondroitin sulfuric acid, aluminum zirconium glycine complex, and acid-alkali neutral salts used in cosmetic formulations.

### • Antioxidants

The antioxidants are not particularly limited. Examples include carotenoids, ascorbic acid and salts thereof, ascorbyl stearate, tocophenol, tocophenol acetate, tocopherol, p-t-butylphenol, butylhydroxyanisole, dibutylhydroxytoluene, phytic acid, ferulic acid, thiotaurine, hypotaurine, sulfite salts, erythorbic acid and salts thereof, chlorogenic acid, epicatechin, epigallocatechin, epigallocatechin gallate, apigenin, kaempferol, myricetin, and quercetin. The antioxidants may be used singly, or two or more may be used in combination.

### • pH Adjusters

Examples of the pH adjusters include lactic acid, citric acid, glycolic acid, succinic acid, tartaric acid, dl-malic acid, potassium carbonate, sodium hydrogen carbonate, and ammonium hydrogen carbonate.

### • Chelating Agents

Examples of the chelating agents include alanine, sodium edetate, sodium polyphosphate, sodium metaphosphate, and phosphoric acid.

### • Algefacients

Examples of the algefacients include L-menthol and camphor.

### • Anti-Inflammatory Agents

Examples of the anti-inflammatory agents include allantoin, glycyrrhizic acid and salts thereof, glycyrrhetinic acid and stearyl glycyrrhetinate, tranexamic acid, and azulene.

### • Skin Beautifying Ingredients

Examples of the skin beautifying ingredients include whitening agents, such as placenta extract, arbutin, glutathione, and Saxifraga Sarmentosa extract; cell activators, such as royal jelly, photosensitizers, cholesterol derivatives, and calf blood extract; skin roughness improvers; circulation promoting ingredients, such as nonylic acid vanillylamide, benzyl nicotinate, β-butoxyethyl nicotinate, capsaicin, zingerone, cantharis tincture, ichthammol, caffeine, tannic acid, α-borneol, tocopherol nicotinate, inositol hexanicotinate, cyclandelate, cinnarizine, tolazoline, acetylcholine, verapamil, cepharanthine, and γ-oryzanol; skin astringents, such as zinc oxide and tannic acid; and antiseborrheic agents, such as sulfur and thianthol.

### • Vitamins

Examples of the vitamins include vitamins A, such as vitamin A oils, retinol, retinyl acetate, and retinyl palmitate; vitamins B, including vitamins B₂, such as riboflavin, riboflavin butyrate, and flavin adenine nucleotide, vitamins B₆, such as pyridoxine hydrochloride, pyridoxine dioctanoate, and pyridoxine tripalmitate, vitamin B₁₂ and derivatives thereof, and vitamin B₁₅ and derivatives thereof; vitamins C, such as L-ascorbic acid, L-ascorbyl dipalmitate, sodium L-ascorbyl-2-sulfate, and dipotassium L-ascorbyl diphosphate; vitamins D, such as ergocalciferol and cholecalciferol; vitamins E, such as α-tocopherol, β-tocopherol, γ-tocopherol, dl-α-tocopherol acetate, dl-α-tocopherol nicotinate, and dl-α-tocopherol succinate; nicotinic acids, such as nicotinic acid, benzyl nicotinate, and nicotinamide; vitamin H; vitamin P; pantothenic acids, such as calcium pantothenate, D-pantothenyl alcohol, pantothenyl ethyl ether, and acetylpantothenyl ethyl ether; and biotin.

### • Amino Acids

Examples of the amino acids include glycine, valine, leucine, isoleucine, serine, threonine, phenylalanine, arginine, lysine, aspartic acid, glutamic acid, cystine, cysteine, methionine, and tryptophan.

### • Nucleic Acids

Examples of the nucleic acids include deoxyribonucleic acid.

### • Hormones

Examples of the hormones include estradiol and ethenyl estradiol.

### • Clathrate Compounds

Examples of the clathrate compounds include cyclodextrin.

In the present invention, the form or shape of the cosmetic is not particularly limited and may be, for example, powder, liquid, or solid. The cosmetic may be any of aqueous emulsion, oil emulsion, water-in-oil emulsion, oil-in-water emulsion, non-aqueous emulsion, or multiphase emulsion, such as W/O/W or O/W/O. Examples of the main forms of the cosmetics according to the present invention include liquids, creams, aerosols, ointments, emulsified solids, sticks, and emulsified sticks.

The use applications of the cosmetics are not particularly limited and include, for example, skin care cosmetics, hair cosmetics, makeup cosmetics, ultraviolet-protecting cosmetics, and antiperspirants. Examples of the skin care cosmetics include lotions, milky lotions, creams, cleansing products, packs, oil liquids, massage products, beauty essences, beauty oils, hand creams, lip creams, and wrinkle concealer skin care cosmetics. Examples of the hair cosmetics include shampoos, conditioners, treatments, hair creams, cuticle coats, and styling agents. Examples of the makeup cosmetics include makeup bases, concealers, face powders, powder foundations, liquid foundations, cream foundations, oil foundations, cheek blushers, eye shadows, mascaras, eyeliners, eyebrows, lip cosmetics, and nail products. Examples of the ultraviolet-protecting cosmetics include sunblock (sunscreen) oils, sunblock milky lotions, sunblock creams, and sunblock lotions.

### EXAMPLES

Hereinafter, the present invention will be described in detail by presenting Examples and Comparative Examples. However, the present invention is not limited to the following Examples. In the following Synthesis Examples, ¹H-NMR was performed using Avance III 400 manufactured by Brucker, and ²⁹Si-NMR was made with ECX-500 manufactured by JEOL Ltd. The purity is the area% in a chromatogram measured by gas chromatography. The kinematic viscosity is a value measured at 25°C using Cannon-Fenske viscometer described in JIS Z8803: 2011. The amounts described with product names are the amounts of the products added.

### [Synthesis Example 1] 1,5-Diethyl-3-ethyldimethylsiloxy-1,1,3,5,5-pentamethyltrisiloxane

A 1 L four-necked flask was charged with 580 g of an organohydrogenpolysiloxane (purity: 100%) represented by formula (4) below and 0.6 g of a 0.5 wt% toluene solution of chloroplatinic acid-vinylsiloxane complex.

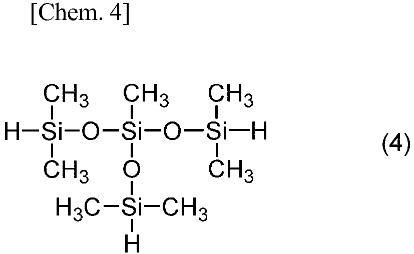

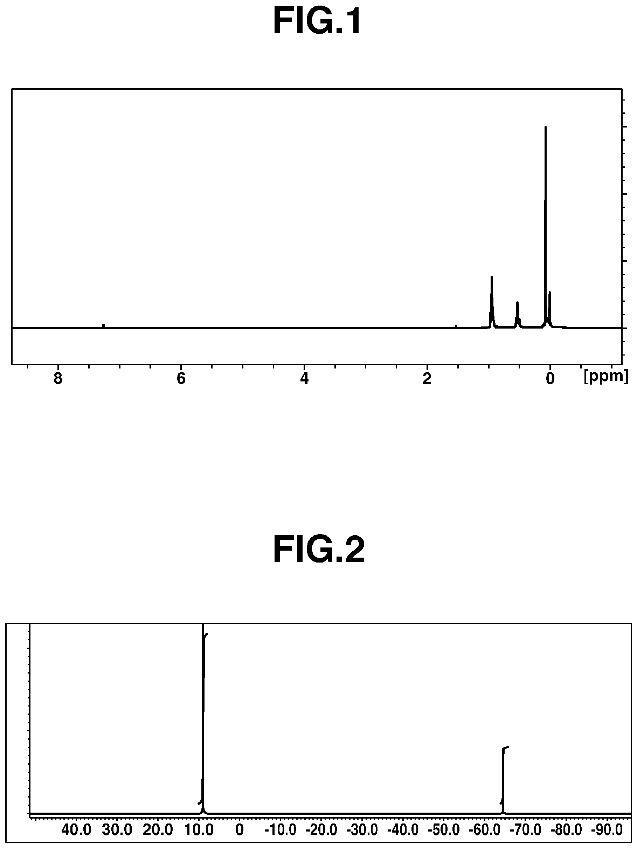

Ethylene gas was blown into the liquid while performing stirring at 40 to 50°C, and the hydrosilylation reaction was carried out while maintaining the temperature at 60 to 70°C. The reaction was followed by gas chromatography. The reaction was terminated when the ratio of the product reached 95% or more. Distillation was performed under reduced pressure (oil bath temperature: 90-130°C, 1-30 hPa) to give 630 g of a colorless transparent liquid fraction. ¹H-NMR and ²⁹Si-NMR spectral analyses of the fraction showed that the target organopolysiloxane (1,5-diethyl-3-ethyldimethylsiloxy-1,1,3,5,5-pentamethyltrisiloxane) having the structure illustrated by formula (5) below was obtained with a purity of 100% and a yield of 83%. The kinematic viscosity was 2.5 mm²/s, and the boiling point was 113°C/10 hPa. The ¹H-NMR spectrum chart is illustrated in FIG. 1, and the ²⁹Si-NMR spectrum chart in FIG. 2.

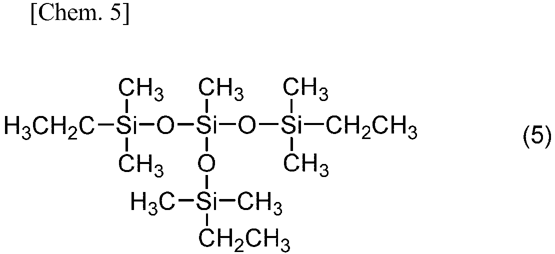

### [Synthesis Example 2] 1,5-Diethyl-3-trimethylsiloxy-1,1,3,5,5-pentamethyltrisiloxane

A 1 L four-necked flask was charged with 490 g of an organohydrogenpolysiloxane (purity: 72%) represented by formula (6) below and 0.5 g of a 0.5 wt% toluene solution of chloroplatinic acid-vinylsiloxane complex.

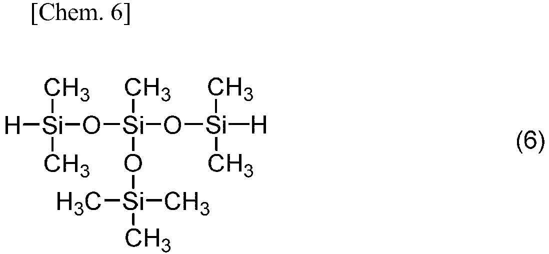

Ethylene gas was blown into the liquid while performing stirring at 40 to 50°C, and the hydrosilylation reaction was carried out while maintaining the temperature at 60 to 70°C. The reaction was followed by gas chromatography. The reaction was terminated when the ratio of the product reached 95% or more. Distillation was performed under reduced pressure (oil bath temperature: 90-130°C, 1-30 hPa) to give 153 g of a colorless transparent liquid fraction. ¹H-NMR and ²⁹Si-NMR spectral analyses of the fraction showed that the target organopolysiloxane (1,5 -diethyl-3 -trimethylsiloxy-1,1,3,5,5 -pentamethyltrisiloxane) having the structure illustrated by formula (7) below was obtained with a purity of 100% and a yield of 36%. The kinematic viscosity was 2.2 mm²/s, and the boiling point was 97°C/10 hPa.

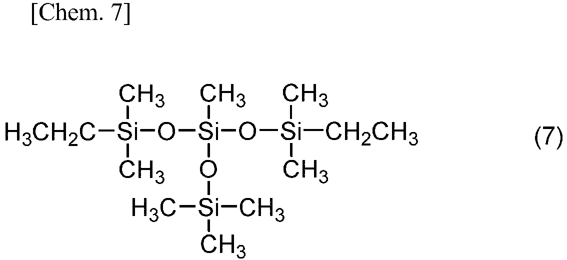

### [Test of Compatibility with Oil Ingredients That Are Solid at 25°C and Crystallization Test]

The branched organopolysiloxane obtained in Synthesis Example 2 and other organopolysiloxanes were tested as described below to evaluate the compatibility with oil ingredients that were solid at 25°C and to evaluate the influence on crystallization. The evaluation results are described in Table 1.

### (Evaluation Methods)

An oil ingredient that was solid at 25°C and the branched organopolysiloxane obtained in Synthesis Example 2 or other organopolysiloxane were collected in a weight ratio of 1:9 and were heated at 90°C for 20 minutes. The state of dissolution was visually examined and was evaluated based on the following criteria.
Uniformly dissolved: ⊚
Uniformly dispersed: ○
Separated: ×

Subsequently, each of the heated mixture liquids was added to a jar container and was allowed to stand at 25°C for 2 days to solidify. The rheometer hardness of the solids was measured. The measurement was performed three times for each solid using rheometer RT2002D·D (manufactured by RHEOTECH) under the conditions where RANGE: 200 g, adapter: 3 mm diameter (rod type), sample stage speed: 5 cm/min, and penetration width: 1 cm. The results of the three measurements were averaged. The higher the value, the higher the hardness (unit: g).

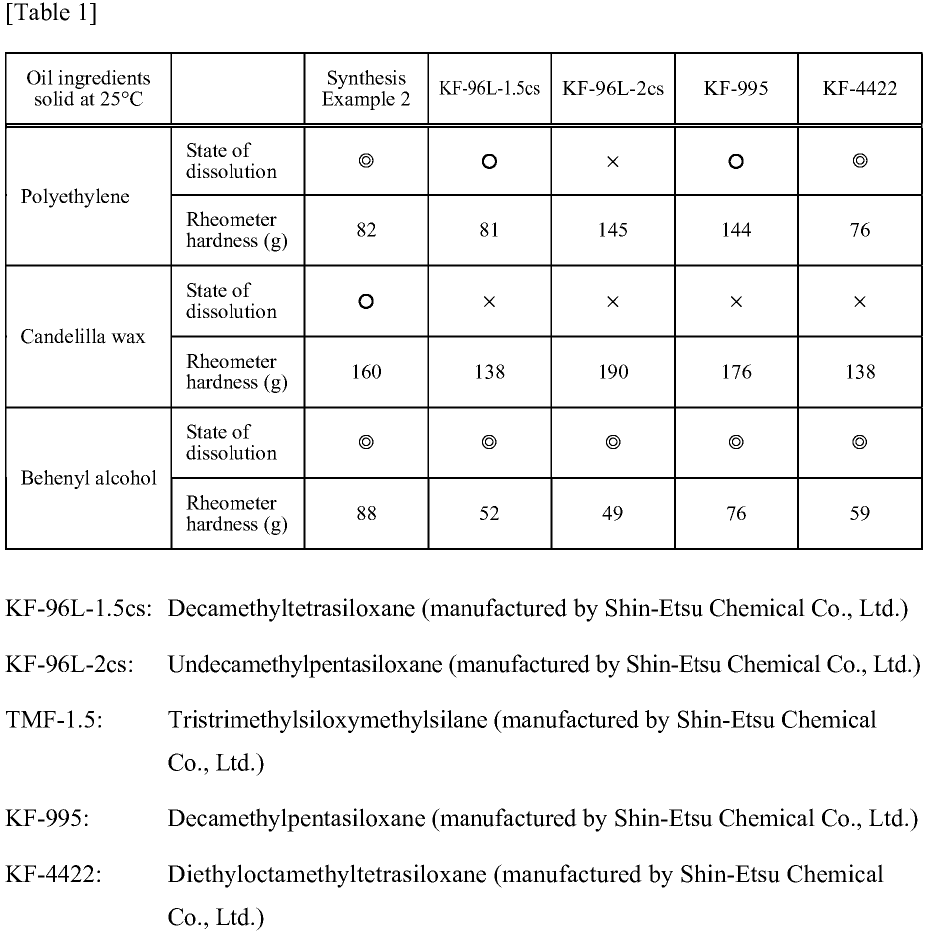

As described in Table 1, the branched organopolysiloxane of the present invention was well dissolved or uniformly dispersed in the solid oil ingredients. In particular, the branched organopolysiloxane of the present invention was uniformly dispersed in candelilla wax, whereas the other silicon compounds were separated. Furthermore, the branched organopolysiloxane of the present invention was shown to have higher compatibility with polyethylene than the linear dimethylpolysiloxanes. Furthermore, it has been shown that the blend composition can be solidified by cooling of the hot composition.

In the preparation of a blend composition using an oil that is highly compatible with a solid oil ingredient, such as wax, it is generally known that cooling of a thermal melt gives a solid composition having low hardness. Even when the ingredients are uniformly dissolved or dispersed when heated, it is sometimes difficult that the composition that has been cooled attains a hardness enough to give a cosmetic with excellent over-time stability. The branched organopolysiloxane of the present invention was well dissolved or uniformly dispersed in the solid oil ingredients and the resultant compositions, after being cooled, had high hardness as compared to the other silicon compounds.

### [Test of Compatibility with Ethylhexyl Methoxycinnamate]

The compatibility of the branched organopolysiloxanes obtained in Synthesis Examples 1 and 2 and other organopolysiloxanes with an organic ultraviolet absorber was evaluated by the method described below. The evaluation results are described in Table 2.

### (Evaluation Method)

Ethylhexyl methoxycinnamate (also written as OMC) and the organopolysiloxane (also written as Sx) were collected in a weight ratio described in Table 2 and were mixed together by shaking at room temperature. The state of dissolution was examined the next day. The state of dissolution is indicated by ○: dissolved and ×: separated.

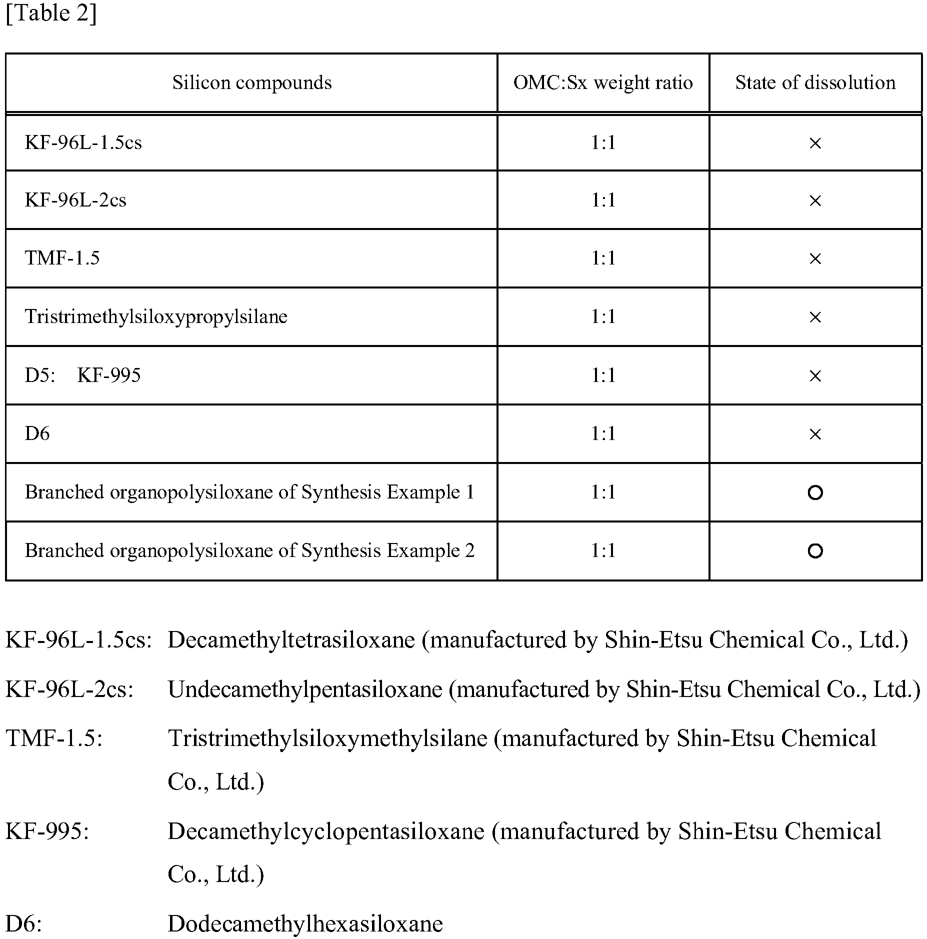

As described in Table 2, the branched organopolysiloxanes obtained in Synthesis Examples 1 and 2 of the present invention were shown to have higher compatibility with ethylhexyl methoxycinnamate than the other silicon compounds.

Hereinafter, the present invention will be described in detail by presenting Examples and Comparative Examples of exemplary cosmetic formulations. However, the present invention is not limited to these Examples. The amounts are indicated in % (wt%) unless otherwise specified.

### [Examples 1 and 2 and Comparative Examples 1 to 3] W/O sunscreens

W/O sunscreens were prepared in accordance with the production method described below using the branched organopolysiloxanes from Synthesis Examples 1 and 2 in Examples 1 and 2, and without using these branched organopolysiloxanes in Comparative Examples 1 to 3. The compositions of Examples 1 and 2 and Comparative Examples 1 to 3 are described in Table 3.

**[Table 3]**

| | | | Example 1 | Example 2 | Comp. Example 1 | Comp. Example 2 | Comp. Example 3 |
|---|---|---|---|---|---|---|---|
| 1 | Branched organopolysiloxane of Synthesis Example 1 | | 12.0 | - | - | - | - |
| 2 | Branched organopolysiloxane of Synthesis Example 2 | | - | 12.0 | - | - | - |
| 3 | KF-995 | | - | - | 12.0 | - | - |
| | | Decamethylcyclopentasiloxane (*1) | | | | | |
| 4 | TMF-1.5 | | - | - | - | 12.0 | - |
| | | Tristrimethylsiloxymethylsilane (*2) | | | | | |
| 5 | KF-56A | | 2.0 | 2.0 | 2.0 | 2.0 | 14.0 |
| | | Diphenylsiloxyphenyl trimethicone (*3) | | | | | |
| 6 | Ethylhexyl methoxycinnamate | | 7.5 | 7.5 | 7.5 | 7.5 | 7.5 |
| 7 | Hexyl diethylaminohydroxybenzoylbenzoate | | 2.0 | 2.0 | 2.0 | 2.0 | 2.0 |
| 8 | Octocrylene | | 2.5 | 2.5 | 2.5 | 2.5 | 2.5 |
| 9 | Ethylhexyl salicylate | | 1.0 | 1.0 | 1.0 | 1.0 | 1.0 |
| 10 | Crosslinked polyether-modified silicone mixture (*4) | | 3.5 | 3.5 | 3.5 | 3.5 | 3.5 |
| 11 | Crosslinked phenyl-modified silicone mixture (*5) | | 3.0 | 3.0 | 3.0 | 3.0 | 3.0 |
| 12 | Alkyl polyether co-modified silicone (*6) | | 1.5 | 1.5 | 1.5 | 1.5 | 1.5 |
| 13 | 1,3-Butylene glycol | | 3.0 | 3.0 | 3.0 | 3.0 | 3.0 |
| 14 | Sodium citrate | | 0.5 | 0.5 | 0.5 | 0.5 | 0.5 |
| 15 | Sodium chloride | | 0.5 | 0.5 | 0.5 | 0.5 | 0.5 |
| 16 | Ethanol | | 5.0 | 5.0 | 5.0 | 5.0 | 5.0 |
| 17 | Purified water | | 56.0 | 56.0 | 56.0 | 56.0 | 56.0 |
| Total | | | 100.0 | 100.0 | 100.0 | 100.0 | 100.0 |

| | | | | | | | |
|---|---|---|---|---|---|---|---|
| (* 1) Decamethylcyclopentasiloxane (manufactured by Shin-Etsu Chemical Co., Ltd.) (*2) Tristrimethylsiloxymethylsilane (INCI: Methyltrimethicone) (manufactured by Shin-Etsu Chemical Co., Ltd.) (*3) Diphenylsiloxyphenyl trimethicone (manufactured by Shin-Etsu Chemical Co., Ltd.) (*4) Mixture of 70-80% dimethicone + 20-30% (dimethicone/(PEG-10/15)) crosspolymer (manufactured by Shin-Etsu Chemical Co., Ltd.) (*5) Mixture of 80-85% diphenylsiloxyphenyl trimethicone + 15-20% crosslinked phenyl-modified dimethylpolysiloxane (manufactured by Shin-Etsu Chemical Co., Ltd.) (*6) Cetyl PEG/PPG-10/1 dimethicone (manufactured by Shin-Etsu Chemical Co., Ltd.) | | | | | | | |

### [Production Method]

The ingredients 1 to 12 were mixed together uniformly by stirring. Separately, the ingredients 13 to 16 were uniformly dissolved into the ingredient 17, and the mixture was gently added to the mixture of the ingredients 1 to 12 described above. The resultant mixture was stirred to form an emulsion, which was then loaded into a predetermined container. A W/O sunscreen was thus obtained.

The W/O sunscreens obtained were used by a panel often female experts to test (1) feel (light feel), (2) spreadability, (3) film uniformity, and (4) durability of the cosmetic film according to the ratings below. The scores were averaged and the results were evaluated based on the criteria below. Furthermore, the cosmetics were observed after being allowed to stand at 40°C for one month (5) and after being allowed to stand at 5°C for one month (6). The evaluation results are described in Table 4.

### (Ratings)

| | |
|---|---|
| 5 Points: | Very good |
| 4 Points: | Good |
| 3 Points: | Fair |
| 2 Points: | Rather poor |
| 1 Point: | Poor |

### (Evaluation Criteria)

| | |
|---|---|
| ⊚: | The average score is 4.5 points or more. |
| ○: | The average score is 3.5 points or more and less than 4.5 points. |
| Δ: | The average score is 2.5 points or more and less than 3.5 points. |
| ×: | The average score is 1.5 points or more and less than 2.5 points. |
| ××: | The average score is less than 1.5 points. |

**[Table 4]**

| No. | Evaluation items | Example 1 | Example 2 | Comparative Example 1 | Comparative Example 2 | Comparative Example 3 |
|---|---|---|---|---|---|---|
| (1) | Feel (light feel) | ○ | ○ | ○ | ○ | × |
| (2) | Spreadability | ⊚ | ⊚ | ⊚ | ○ | ⊚ |
| (3) | Film uniformity | ⊚ | ⊚ | ⊚ | Δ | ○ |
| (4) | Cosmetic film durability | ○ | ○ | ○ | Δ | Δ |
| (5) | State of the cosmetic (40°C) | Stable | Stable | Increased viscosity | Separated | Stable |
| (6) | State of the cosmetic (5°C) | Stable | Stable | Separated | Separated | Stable |

As clear from Table 4, the sunscreens of Examples 1 and 2 as compared to Comparative Examples 1 to 3 were demonstrated to have a light and oil-free feel, spread well, and form a highly water-repellent and uniform film, that is, a long-lasting and uniform cosmetic film. The sunscreens were also shown to have excellent storage stability.

### [Examples 3 and 4 and Comparative Examples 4 and 5] Lipsticks

Lipsticks were produced in accordance with the production method described below using the branched organopolysiloxanes from Synthesis Examples 1 and 2 in Examples 3 and 4, and without using these branched organopolysiloxanes in Comparative Examples 4 and 5. The compositions of Examples 3 and 4 and Comparative Examples 4 and 5 are described in Table 5.

**[Table 5]**

| Composition (%) | | Example 3 | Example 4 | Comp. Example 4 | Comp. Example 5 |
|---|---|---|---|---|---|
| 1 | Siloxane of Synthesis Example 1 | 28.8 | - | - | - |
| 2 | Siloxane of Synthesis Example 2 | - | 28.8 | - | - |
| 3 | Decamethylcyclopentasiloxane (*1) | - | | 28.8 | - |
| 4 | Dimethicone 2 cs (*2) | - | - | | 28.8 |
| 5 | Isotridecyl isononanoate | 10 | 10 | 10 | 10 |
| 6 | Diisostearyl malate | 5 | 5 | 5 | 5 |
| 7 | Candelilla wax | 10 | 10 | 10 | 10 |
| 8 | Polyethylene | 7 | 7 | 7 | 7 |
| 9 | Microcrystalline wax | 2 | 2 | 2 | 2 |
| 10 | Trimethylsiloxysilicate solution (*3) | 20 | 20 | 20 | 20 |
| 11 | Crosslinked alkyl-modified silicone mixture (*4) | 5 | 5 | 5 | 5 |
| 12 | Silicone branched alkylpolyglycerin co-modified silicone (*5) | 3 | 3 | 3 | 3 |
| 13 | Colorant | 1.2 | 1.2 | 1.2 | 1.2 |
| 14 | Mica | 8 | 8 | 8 | 8 |
| Total | | 100.0 | 100.0 | 100.0 | 100.0 |

| | | | | | |
|---|---|---|---|---|---|
| (*1) Decamethylcyclopentasiloxane (manufactured by Shin-Etsu Chemical Co., Ltd.) (*2) Dimethicone (2 cs) (manufactured by Shin-Etsu Chemical Co., Ltd.) (*3) 50% Cyclopentasiloxane solution of trimethylsiloxysilicate (manufactured by Shin-Etsu Chemical Co., Ltd.) (*4) Mixture of 65-75% triethylhexanoin + 25-35% alkyl branched crosslinked dimethylpolysiloxane (manufactured by Shin-Etsu Chemical Co., Ltd.) (*5) Lauryl polyglyceryl-3 polydimethylsiloxyethyl dimethicone (manufactured by Shin-Etsu Chemical Co., Ltd.) | | | | | |

### [Production Method]

A: The ingredient 13 was mixed with a portion of the ingredient 5 and the mixture was dispersed with a roll mill. The dispersion obtained was mixed with the ingredients 1 to 4, the remainder of the ingredient 5, and the ingredients 6 to 14 while performing heating to give a composition.
B: The mixture obtained in A was poured into a mold and was solidified by cooling. A lipstick was thus obtained.

The lipsticks obtained were used by a panel of eight female experts to test (1) spreadability, (2) film uniformity, and (3) cosmetic durability according to the ratings below. The scores were averaged and the results were evaluated based on the criteria below. Furthermore, the cosmetics were observed after being allowed to stand at 40°C for one month (4). The evaluation results are described in Table 6.

### (Ratings)

| | |
|---|---|
| 5 Points: | Very good |
| 4 Points: | Good |
| 3 Points: | Fair |
| 2 Points: | Rather poor |
| 1 Point: | Poor |

### (Evaluation Criteria)

| | |
|---|---|
| ⊚: | The average score is 4.5 points or more. |
| ○: | The average score is 3.5 points or more and less than 4.5 points. |
| Δ: | The average score is 2.5 points or more and less than 3.5 points. |
| ×: | The average score is 1.5 points or more and less than 2.5 points. |
| ××: | The average score is less than 1.5 points. |

**[Table 6]**

| No. | Evaluation items | Example 3 | Example 4 | Comparative Example 4 | Comparative Example 5 |
|---|---|---|---|---|---|
| (1) | Spreadability | ○ | ⊚ | ○ | ○ |
| (2) | Film uniformity | ⊚ | ⊚ | Δ | ⊚ |
| (3) | Cosmetic durability | ⊚ | ○ | ○ | Δ |
| (4) | State of the cosmetic (40°C) | Stable | Stable | Separated | Separated |

As clear from Table 6, the lipsticks of Examples 3 and 4 were demonstrated to spread well and form a uniform and highly water-repellent film, that is, a long-lasting film. The lipsticks were also shown to have excellent storage stability.

### [Example 5] Sunscreen milky lotion (shaking)

| Composition | | % |
|---|---|---|
| 1. | KF-995 (*1) | 22.5 |
| 2. | Siloxane of Synthesis Example 2 | 9 |
| 3. | KSG-18A (*2) | 3 |
| 4. | KF-6038 (*3) | 2 |
| 5. | Ethylhexyl methoxycinnamate | 7.5 |
| 6. | Hexyl diethylaminohydroxybenzoylbenzoate | 1 |
| 7. | Octocrylene | 2.5 |
| 8. | Disteardimonium hectorite | 1 |
| 9. | KP-545 (*4) | 2 |
| 10. | KSP-105 (*5) | 0.5 |
| 11. | SPD-T5 (*6) | 5 |
| 12. | SPD-Z5 (*7) | 10 |
| 13. | BG | 3 |
| 14. | Sodium citrate | 0.2 |
| 15. | Sodium chloride | 0.5 |
| 16. | Ethanol | 5 |
| 17. | Water | 25.3 |
| Total | | 100.0 |

| | | |
|---|---|---|
| [0121] (*1) Cyclopentasiloxane (manufactured by Shin-Etsu Chemical Co., Ltd.) (*2) Mixture of 80-90% diphenylsiloxyphenyl trimethicone + 10-20% (dimethicone/phenylvinyl dimethicone) crosspolymer (manufactured by Shin-Etsu Chemical Co., Ltd.) (*3) Lauryl PEG-9 polydimethylsiloxyethyl dimethicone (manufactured by Shin-Etsu Chemical Co., Ltd.) (*4) Solution of 70% cyclopentasiloxane + 30% (acrylates/dimethicone) copolymer (manufactured by Shin-Etsu Chemical Co., Ltd.) (*5) (Vinyl dimethicone/methicone silsesquioxane) crosspolymer (manufactured by Shin-Etsu Chemical Co., Ltd.) (*6) 40% Cyclopentasiloxane dispersion of fine titanium oxide particles (manufactured by Shin-Etsu Chemical Co., Ltd.) (*7) 60% Cyclopentasiloxane dispersion of fine zinc oxide particles (manufactured by Shin-Etsu Chemical Co., Ltd.) | | |

### [Production Method]

A: The ingredients 1 to 9 were mixed uniformly.
B: The ingredient 10 was added to the mixture obtained in A and was uniformly dispersed therein.
C: The ingredients 13 to 16 were added to the ingredient 17 and were dissolved therein.
D: The mixture obtained in C was gradually added to the mixture obtained in B to form an emulsion. The ingredients 11 and 12 were added. A sunscreen milky lotion was thus obtained.

The sunscreen milky lotion obtained as described above spread lightly, left the skin dry without stickiness, and was free from changes due to temperature or aging, thus being shown to have excellent usability and stability.

### [Example 6] Sunscreen cream

| Composition | | % |
|---|---|---|
| 1. | Siloxane of Synthesis Example 1 | 30 |
| 2. | Squalane | 3 |
| 3. | KF-6105 (*1) | 4 |
| 4. | Ethylhexyl methoxycinnamate | 7.5 |
| 5. | t-Butylmethoxydibenzoylmethane | 3 |
| 6. | Polysilicone-15 | 1 |
| 7. | Distearyldimonium chloride | 1 |
| 8. | Tocopherol acetate | 0.1 |
| 9. | Ethanol | 1 |
| 10. | Sodium citrate | 0.5 |
| 11. | Magnesium sulfate | 0.5 |
| 12. | Preservative | 0.3 |
| 13. | Water | 48.1 |
| Total | | 100.0 |

| | | |
|---|---|---|
| (* 1) Lauryl polyglyceryl-3 polydimethylsiloxyethyl dimethicone (manufactured by Shin-Etsu Chemical Co., Ltd.) | | |

### [Production Method]

A: The ingredients 1 to 8 were uniformly mixed.
B: The ingredients 9 to 12 were uniformly dissolved into the ingredient 13.
C: The mixture obtained in B was gradually added to the mixture obtained in A while performing stirring to form an emulsion. A sunscreen cream was thus obtained.

The sunscreen cream obtained as described above had a fine texture, spread lightly, gave a moist and dewy feel, and was free from stickiness and caught no sand, thus being shown to have very good usability. Furthermore, the sunscreen cream was free from changes due to temperature or aging, thus being shown to have excellent stability.

### [Example 7] Sunscreen cream

| Composition | | % |
|---|---|---|
| 1. | Siloxane of Synthesis Example 2 | 16 |
| 2. | KSG-310 (*1) | 3.5 |
| 3. | KSG-18A (*2) | 3 |
| 4. | KF-6048 (*3) | 1.5 |
| 5. | KF-7312J (*4) | 3 |
| 6. | Ethylhexyl methoxycinnamate | 7.5 |
| 7. | t-Butylmethoxydibenzoylmethane | 3 |
| 8. | Ethylhexyl salicylate | 3 |
| 9. | BG | 5 |
| 10. | Sodium citrate | 0.5 |
| 11. | Sodium chloride | 1 |
| 12. | Preservative | 0.3 |
| 13. | Water | 52.7 |
| Total | | 100.0 |

| | | |
|---|---|---|
| (* 1) Mixture of 65-75% mineral oil + 25-35% (PEG-15/lauryl dimethicone) crosspolymer (manufactured by Shin-Etsu Chemical Co., Ltd.) (*2) Mixture of 80-90% diphenylsiloxyphenyl trimethicone + 10-20% (dimethicone/phenylvinyl dimethicone) crosspolymer (manufactured by Shin-Etsu Chemical Co., Ltd.) (*3) Cetyl PEG/PPG-10/1 dimethicone (manufactured by Shin-Etsu Chemical Co., Ltd.) (*4) 50% Cyclopentasiloxane solution of trimethylsiloxysilicate (manufactured by Shin-Etsu Chemical Co., Ltd.) | | |

### [Production Method]

A: The ingredients 1 to 8 were uniformly mixed.
B: The ingredients 9 to 12 were uniformly dissolved into the ingredient 13.
C: The mixture obtained in B was gradually added to the mixture obtained in A while performing stirring to form an emulsion. A sunscreen cream was thus obtained.

The sunscreen cream obtained as described above spread lightly, gave a dewy feel without stickiness, and formed a uniform and highly water-repellent film, thus offering long-lasting cosmetic and ultraviolet protective effects. Furthermore, the sunscreen cream was free from changes due to temperature or aging. Thus, the sunscreen cream was shown to have excellent usability and stability.

### [Example 8] Non-aqueous mousse foundation

| Composition | | % |
|---|---|---|
| 1. | KSG-240 (*1) | 18 |
| 2. | KF-96A-6CS (*2) | 1 |
| 3. | Siloxane of Synthesis Example 2 | 11 |
| 4. | Ethylhexyl methoxycinnamate | 5 |
| 5. | Jojoba oil | 1 |
| 6. | Silylated silicic anhydride (*3) | 0.75 |
| 7. | KTP-09R (*4) | 0.2 |
| 8. | KTP-09Y (*4) | 1 |
| 9. | KTP-09B (*4) | 0.02 |
| 10. | KTP-09W (*4) | 5 |
| 11. | KF-9909 (*5) treated talc | 11.55 |
| 12. | KF-7312J (*6) | 4 |
| 13. | KF-995 (*7) | 25.28 |
| 14. | KSP-411 (*8) | 6 |
| 15. | KMP-590 (*9) | 3 |
| 16. | Spherical polyalkyl methacrylate | 7 |
| 17. | Antioxidant | 0.2 |
| Total | | 100.0 |

| | | |
|---|---|---|
| (*1) Mixture of 75-85% cyclopentasiloxane + 15-25% (dimethicone/(PEG-10/15) crosspolymer (manufactured by Shin-Etsu Chemical Co., Ltd.) (*2) Dimethicone (6 cs) (manufactured by Shin-Etsu Chemical Co., Ltd.) (*3) Surface-hydrophobized fumed silica: Aerosil R-972 (manufactured by Nippon Aerosil Co., Ltd.) (*4) KF-9909 treated colored inorganic pigments, W: white, R: red, Y: yellow, B: black (manufactured by Shin-Etsu Chemical Co., Ltd.) (*5) Triethoxysilylethylpolydimethylsiloxyethylhexyldimethicone (manufactured by Shin-Etsu Chemical Co., Ltd.) (*6) 50% Cyclopentasiloxane solution of trimethylsiloxysilicate (manufactured by Shin-Etsu Chemical Co., Ltd.) (*7) Cyclopentasiloxane (manufactured by Shin-Etsu Chemical Co., Ltd.) (*8) Polysilicone-1 crosspolymer (manufactured by Shin-Etsu Chemical Co., Ltd.) (*9) Polymethylsilsesquioxane (manufactured by Shin-Etsu Chemical Co., Ltd.) | | |

### [Production Method]

The ingredients 1 to 10 were uniformly mixed by roller treatment. The ingredients 11 to 17 were added to the mixture and the resultant mixture was mixed uniformly to give a non-aqueous mousse foundation.

The foundation obtained as described above had a mousse-like appearance, was firm, spread lightly, had an excellent feeling on use without stickiness or oily feel, and offered very long-lasting cosmetic effects. Furthermore, the foundation was free from oil bleeding or the like due to temperature or aging, and had excellent stability.

### [Example 9] Non-aqueous eye wrinkle cream

| Composition | | % |
|---|---|---|
| 1. | KSG-210 (*1) | 5 |
| 2. | KSG-41A (*2) | 7 |
| 3. | KSG-15 (*3) | 55 |
| 4. | KF-4422 (*4) | 8 |
| 5. | Siloxane of Synthesis Example 1 | 6 |
| 6. | Jojoba oil | 2 |
| 7. | KSP-101 (*5) | 12 |
| 8. | KF-9028 (*6) | 5 |
| Total | | 100.0 |

| | | |
|---|---|---|
| (* 1) Mixture of 70-80% dimethicone + 20-30% (dimethicone/(PEG-10/15)) crosspolymer (manufactured by Shin-Etsu Chemical Co., Ltd.) (*2) Mixture of 70-80% mineral oil + 20-30% (vinyl dimethicone/lauryl dimethicone) crosspolymer (manufactured by Shin-Etsu Chemical Co., Ltd.) (*3) Mixture of 90-96% cyclopentasiloxane + 4-10% (dimethicone/vinyl dimethicone) crosspolymer (manufactured by Shin-Etsu Chemical Co., Ltd.) (*4) Ethylmethicone (manufactured by Shin-Etsu Chemical Co., Ltd.) (*5) (Vinyl dimethicone/methicone silsesquioxane) crosspolymer (manufactured by Shin-Etsu Chemical Co., Ltd.) (*6) 20% Cyclopentasiloxane solution of dimethicone (high degree of polymerization) (manufactured by Shin-Etsu Chemical Co., Ltd.) | | |

### [Production Method]

### A: The ingredients 1 to 8 were mixed uniformly to give an eye wrinkle cream.

The eye wrinkle cream obtained as described above spread lightly, had no stickiness or oily feel, and offered a moist and comfortable feel to the skin. Furthermore, the eye wrinkle cream was free from changes due to temperature or aging, thus being shown to have excellent stability.

### [Example 10] Wash-off pack cosmetic

| Composition | | % |
|---|---|---|
| 1. | KF-96A-6CS (*1) | 3 |
| 2. | Siloxane of Synthesis Example 1 | 3 |
| 3. | KF-6100 (*2) | 2 |
| 4. | Kaolin | 30 |
| 5. | Carbomer | 0.4 |
| 6. | BG | 10 |
| 7. | Glycerin | 20 |
| 8. | Preservative | 0.1 |
| 9. | Fragrance | 0.1 |
| 10. | Water | 31.4 |
| Total | | 100.0 |

| | | |
|---|---|---|
| (*1) Dimethicone (6 cs) (manufactured by Shin-Etsu Chemical Co., Ltd.) (*2) Polyglyceryl-3 disiloxane dimethicone (manufactured by Shin-Etsu Chemical Co., Ltd.) | | |

### [Production Method]

A: The ingredients 1 to 3 and 8 were mixed.
B: The ingredients 5 to 7 and 10 were mixed uniformly, and subsequently the ingredients 4 and 9 were mixed. The resultant mixture was stirred.
C: The mixture obtained in A was added to the mixture obtained in B to form an emulsion. A paste-like wash-off pack cosmetic was thus obtained.

The wash-off pack cosmetic obtained as described above spread lightly during application and offered excellent cleansing effects. After the pack cosmetic was rinsed off, the skin had a moist and smooth feel without stickiness. The pack cosmetic thud had a very good feeling on use and was also shown to have excellent stability.

### [Example 11] Eye color

| Composition | | % |
|---|---|---|
| 1. | TMF-1.5 (*1) | 25.5 |
| 2. | KP-550 (*2) | 20 |
| 3. | KP-561P (*3) | 2 |
| 4. | KSP-441 (*4) | 6 |
| 5. | Isododecane | 3 |
| 6. | Siloxane of Synthesis Example 2 | 5 |
| 7. | Vaseline | 5 |
| 8. | KSG-320 (*5) | 5 |
| 9. | Amorphous silicic anhydride (*6) | 1 |
| 10. | Barium sulfate | 5 |
| 11. | Organic pigment | 0.2 |
| 12. | KTP-09Y (*7) | 1 |
| 13. | KTP-09W (*7) | 1 |
| 14. | KF-9909 (*8) treated, titanated mica | 20 |
| 15. | Tocopherol | 0.2 |
| 16. | Fragrance | 0.1 |
| Total | | 100.0 |

| | | |
|---|---|---|
| (*1) Methyltrimethicone (manufactured by Shin-Etsu Chemical Co., Ltd.) (*2) Solution of 60% isododecane + 40% (acrylates/dimethicone) copolymer (manufactured by Shin-Etsu Chemical Co., Ltd.) (*3) (Acrylates/stearyl acrylate/dimethicone methacrylate) copolymer (manufactured by Shin-Etsu Chemical Co., Ltd.) (*4) Polysilicone-22 (manufactured by Shin-Etsu Chemical Co., Ltd.) (*5) Mixture of 70-80% isododecane + 20-30% (PEG-15/lauryl dimethicone) crosspolymer (manufactured by Shin-Etsu Chemical Co., Ltd.) (*6) Surface-hydrophobized fumed silica: Aerosil R-972 (manufactured by Nippon Aerosil Co., Ltd.) (*7) KF-9909 treated colored inorganic pigments, W: white, Y: yellow (manufactured by Shin-Etsu Chemical Co., Ltd.) (*8) Triethoxysilylethylpolydimethylsiloxyethylhexyldimethicone (manufactured by Shin-Etsu Chemical Co., Ltd.) | | |

### [Production Method]

A: The ingredients 1 to 9 were mixed together to give a uniform dispersion.
B: The ingredients 10 to 16 were added to the mixture obtained in A. The mixture was uniformly dispersed to give an eye color.

The eye color obtained as described above was easy to remove, spread lightly, and did not feel oily or powdery when used. Furthermore, the eye color had good water resistance, water repellency, and perspiration resistance and thus had good durability and was resistant to coming off. Furthermore, the eye color was free from changes due to temperature or aging, thus being shown to have excellent stability.

### [Example 12] Eyeliner

| Composition | | % |
|---|---|---|
| 1. | Siloxane of Synthesis Example 2 | 22 |
| 2. | KF-96A-6CS (*1) | 5 |
| 3. | KSG-42A (*2) | 5 |
| 4. | Jojoba oil | 2 |
| 5. | KF-6038 (*3) | 3 |
| 6. | KF-9901 (*4) treated black iron oxide | 20 |
| 7. | Ethanol | 5 |
| 8. | Preservative | 0.1 |
| 9. | Water | 37.9 |
| Total | | 100.0 |

| | | |
|---|---|---|
| (*1) Dimethicone (6 cs) (manufactured by Shin-Etsu Chemical Co., Ltd.) (*2) Mixture of 75-85% isododecane + 15-25% (vinyl dimethicone/lauryl dimethicone) crosspolymer (manufactured by Shin-Etsu Chemical Co., Ltd.) (*3) Lauryl PEG-9 polydimethylsiloxyethyl dimethicone (manufactured by Shin-Etsu Chemical Co., Ltd.) (*4) Hydrogen dimethicone (manufactured by Shin-Etsu Chemical Co., Ltd.) | | |

### [Production Method]

A: The ingredients 1 to 5 were mixed under heating conditions, and the ingredient 6 was added. The mixture was uniformly dispersed.
B: The ingredients 7 to 9 were dissolved under heating conditions.
C: The mixture obtained in B was gradually added to the mixture obtained in A while performing stirring to form an emulsion. An eyeliner was thus obtained.

The eyeliner obtained as described above spread lightly without feeling oily or powdery, gave a dewy and refreshing feeling on use. Furthermore, the eyeliner had good water resistance, water repellency, and perspiration resistance and thus had good durability and was resistant to coming off. Furthermore, the eyeliner was free from changes due to temperature or aging, thus being shown to have excellent stability.

### [Example 13] W/O cleansing cream

| Composition | | % |
|---|---|---|
| 1. | KF-96A-6CS (*1) | 10 |
| 2. | KF-54 (*2) | 15 |
| 3. | Mineral oil | 8 |
| 4. | Isostearic acid | 1 |
| 5. | Siloxane of Synthesis Example 1 | 11 |
| 6. | Dextrin fatty acid ester | 0.8 |
| 7. | KF-6017 (*3) | 4 |
| 8. | Glycerin | 10 |
| 9. | Sodium citrate | 0.2 |
| 10. | Sodium chloride | 1 |
| 11. | Preservative | 0.1 |
| 12. | Fragrance | 0.1 |
| 13. | Purified water | 38.8 |
| Total | | 100.0 |

| | | |
|---|---|---|
| (*1) Dimethicone (6 cs) manufactured by Shin-Etsu Chemical Co., Ltd. (*2) Diphenyl dimethicone manufactured by Shin-Etsu Chemical Co., Ltd. (*3) PEG-10 Dimethicone manufactured by Shin-Etsu Chemical Co., Ltd. | | |

A: The ingredients 1 to 7 were mixed under heating conditions.
B: The ingredients 8 to 11 and 13 were dissolved under heating conditions.
C: While performing stirring, the mixture obtained in B was gradually added to the mixture obtained in A to form an emulsion, which was then cooled. The ingredient 12 was added. A W/O cleansing cream was thus obtained.

The W/O cleansing cream obtained as described above had a fine texture, spread lightly without feeling sticky or oily, gave a moist, dewy, and refreshing feeling on use, and offered high cleansing effects. Furthermore, the W/O cleansing cream was free from changes due to temperature or aging, thus being shown to have excellent stability.

### [Example 14] Lip cream

| Composition | | % |
|---|---|---|
| 1. | Dextrin (palmitate/ethylhexanoate) (*1) | 9 |
| 2. | Siloxane of Synthesis Example 2 | 7 |
| 3. | KP-545 (*2) | 5 |
| 4. | KSG-43 (*3) | 8 |
| 5. | KF-6105 (*4) | 2 |
| 6. | KF-995 (*5) | 46 |
| 7. | 1,3-Butylene glycol | 5 |
| 8. | Purified water | 10.8 |
| 9. | Colorant | 1.2 |
| 10. | Titanium oxide coated mica | 6 |
| Total | | 100.0 |

| | | |
|---|---|---|
| (*1) Rheopearl TT (manufactured by Chiba Flour Milling Co., Ltd.) (*2) Solution of 70% cyclopentasiloxane + 30% (acrylates/dimethicone) copolymer (manufactured by Shin-Etsu Chemical Co., Ltd.) (*3) Mixture of 65-75% triethylhexanoin + 25-35% (vinyl dimethicone/lauryl dimethicone) crosspolymer (manufactured by Shin-Etsu Chemical Co., Ltd.) (*4) Lauryl polyglyceryl-3 polydimethylsiloxyethyl dimethicone (manufactured by Shin-Etsu Chemical Co., Ltd.) (*5) Cyclopentasiloxane (manufactured by Shin-Etsu Chemical Co., Ltd.) | | |

### [Production Method]

A: The ingredient 9 was mixed with a portion of the ingredient 2 and the mixture was dispersed using a roller mill. The resultant dispersion was mixed with the ingredient 1, the remainder of the ingredient 2, and the ingredients 3 to 6 under heating conditions.
B: The ingredients 7 and 8 were heated and were added to the mixture obtained in A to form an emulsion, which was then cooled.
C: The ingredient 10 was added to the emulsion obtained in B. A lip cream was thus obtained.

The lip cream obtained spread lightly, was free from stickiness or oily feel, and formed a long-lasting film on the lips.

### [Example 15] Mascara

| Composition | | % |
|---|---|---|
| 1. | KF-6028 (*1) | 1 |
| 2. | Disteardimonium hectorite | 4 |
| 3. | Isododecane | 39.5 |
| 4. | KP-550 (*2) | 20 |
| 5. | Dextrin palmitate/ethylhexanoate (*3) | 3 |
| 6. | Ceresin | 2.5 |
| 7. | KP-562P (*4) | 2 |
| 8. | Beeswax | 2.5 |
| 9. | Siloxane of Synthesis Example 2 | 5 |
| 10. | Hydrogenated lecithin | 0.5 |
| 11. | Silica | 3 |
| 12. | Talc | 12 |
| 13. | KTP-09B (*5) | 5 |
| Total | | 100.0 |

| | | |
|---|---|---|
| (*1) PEG-9 polydimethylsiloxyethyl dimethicone (manufactured by Shin-Etsu Chemical Co., Ltd.) (*2) Solution of 60% isododecane + 40% (acrylates/dimethicone) copolymer (manufactured by Shin-Etsu Chemical Co., Ltd.) (*3) Rheopearl TT (manufactured by Chiba Flour Milling Co., Ltd.) (*4) (Acrylates/behenyl acrylate/dimethicone methacrylate) copolymer (manufactured by Shin-Etsu Chemical Co., Ltd.) (*5) KF-9909 treated colored inorganic pigment, B: Black (manufactured by Shin-Etsu Chemical Co., Ltd.) | | |

### [Production Method]

A: The ingredients 1 to 3 were mixed uniformly.
B: The ingredients 4 to 10 were dissolved by stirring under heating conditions, and the mixture obtained in A and crushed ingredients 11, 12, and 13 were added. The mixture was mixed uniformly and was then cooled.

The mascara obtained as described above was non-sticky, spread lightly, was easy to apply to the eyelashes, and offered very long-lasting cosmetic effects.

### [Example 16] Oil cleansing product

| Composition | | % |
|---|---|---|
| 1. | Mineral oil | 30 |
| 2. | Isopropyl myristate | 2 |
| 3. | Siloxane of Synthesis Example 1 | 54.9 |
| 4. | PEG-6 diisostearate | 1 |
| 5. | Tocopherol acetate | 0.1 |
| 6. | PEG-20 glyceryl triisostearate | 10 |
| 7. | Glycerin | 1 |
| 8. | Water | 1 |
| Total | | 100.0 |

### [Production Method]

A: The ingredients 1 to 6 were mixed uniformly.
B: The ingredients 7 and 8 were dissolved by stirring. The solution was added to the mixture obtained in A, and the resultant mixture was mixed uniformly to give an oil cleansing product.

The oil cleansing product obtained as described above was non-sticky, spread easily to form a uniform oil film, and had very high cleansing effects.

### [Example 17] Deodorant stick

| | | Composition | % |
|---|---|---|---|
| 1. | Chlorohydroxy Al | | 23 |
| 2. | Siloxane of Synthesis Example 2 | | 36.5 |
| 3. | Stearyl alcohol | | 8 |
| 4. | Talc | | 14.88 |
| 5. | Fragrance | | 0.1 |
| 6. | BHT | | 0.02 |
| 7. | Paraffin (solid) | | 2 |
| 8. | Mineral oil | | 14.5 |
| 9. | KF-6048 (*1) | | 1 |
| | | Total | 100.0 |

| | | | |
|---|---|---|---|
| (*1) Cetyl PEG/PPG-10/1 dimethicone (manufactured by Shin-Etsu Chemical Co., Ltd.) | | | |

### [Production Method]

A: The ingredients 2, 3, and 6 to 9 were dissolved under heating conditions and mixed together.
B: The ingredients 1 and 4 were uniformly mixed with the mixture obtained in A. The resultant mixture was uniformly dispersed and mixed using a mixer.
C: The ingredient 5 was added to the mixture obtained in B and the resultant mixture was mixed uniformly.
D: The mixture obtained in C was poured into a mold and was cooled to solidify.

The deodorant stick obtained as described above was free from an excessively dry or sticky feeling and was excellent in long-lasting deodorizing effects.

### [Example 18] Deodorant spray

| | | Composition | % |
|---|---|---|---|
| 1. | Chlorohydroxy Al | | 30 |
| 2. | Silica | | 15 |
| 3. | Siloxane of Synthesis Example 1 | | 10 |
| 4. | KF-9909 (*1) treated talc | | 14.88 |
| 5. | Fragrance | | 0.1 |
| 6. | BHT | | 0.02 |
| 7. | Zinc oxide | | 5 |
| 8. | Triclosan | | 0.1 |
| 9. | Isopropyl myristate | | 21.9 |
| 10. | KF-6105 (*2) | | 3 |
| | | Total | 100.0 |

| | | | |
|---|---|---|---|
| (* 1) Triethoxysilylethyl polydimethylsiloxyethylhexyl dimethicone (manufactured by Shin-Etsu Chemical Co., Ltd.) (*2) Lauryl polyglyceryl-3 polydimethylsiloxyethyl dimethicone (manufactured by Shin-Etsu Chemical Co., Ltd.) | | | |

### [Production Method]

A: The ingredients 2 to 4 and 6 to 10 were uniformly mixed.
B: The ingredient 1 was uniformly mixed with the mixture obtained in A. The resultant mixture was uniformly dispersed and mixed using a mixer.
C: The ingredient 5 was added to the mixture obtained in B. The resultant mixture was mixed uniformly.
D: A spray container was filled with 10 parts by weight of the mixture obtained in C (stock liquid) and 90 parts by weight of LPG.

The deodorant spray obtained as described above was free from an excessively dry or sticky feeling and was excellent in long-lasting deodorizing effects.

### [Example 19] Non-aqueous deodorant cream

| | | Composition | % |
|---|---|---|---|
| 1. | Trichlorohydrex glycine (Al/zirconium) | | 19 |
| 2. | Siloxane of Synthesis Example 2 | | 30 |
| 3. | KSG-15 (*1) | | 21.5 |
| 4. | KSP-100 (*2) | | 10 |
| 5. | Neopentyl glycol dioctanoate | | 9.68 |
| 6. | Fragrance | | 0.1 |
| 7. | BHT | | 0.02 |
| 8. | Citric acid | | 0.1 |
| 9. | Benzyl alcohol | | 0.1 |
| 10. | Dimethylsilylated silica | | 0.5 |
| 11. | Polyethylene | | 3 |
| 12. | Ceresin | | 6 |
| | | Total | 100.0 |

| | | | |
|---|---|---|---|
| (*1) Mixture of 90-96% cyclopentasiloxane + 4-10% (dimethicone/vinyl dimethicone) crosspolymer (manufactured by Shin-Etsu Chemical Co., Ltd.) (*2) (Vinyl dimethicone/methicone silsesquioxane) crosspolymer (manufactured by Shin-Etsu Chemical Co., Ltd.) | | | |

### [Production Method]

A: The ingredients 2, 3, 5, 7, 9, 11, and 12 were heated and were mixed uniformly.
B: The ingredient 10 was uniformly mixed with the mixture obtained in A. The resultant mixture was uniformly dispersed and mixed using a mixer.
C: The ingredients 1, 4, and 8 were added to the mixture obtained in B. The resultant mixture was mixed uniformly.
D: The ingredient 6 was added to the mixture obtained in C. The resultant mixture was mixed uniformly and was then loaded into a container.

The non-aqueous deodorant cream obtained as described above spread very smoothly and well and was free from an excessively dry or sticky feeling. Furthermore, the non-aqueous deodorant cream was excellent in long-lasting deodorizing effects.

### [Example 20] W/O deodorant cream

| | | Composition | % |
|---|---|---|---|
| 1. | KSG-210 (*1) | | 3 |
| 2. | KSG-15 (*2) | | 2 |
| 3. | KF-6028 (*3) | | 2 |
| 4. | Ethylhexyl palmitate | | 5 |
| 5. | Isopropylmethylphenol | | 0.1 |
| 6. | Chlorohydroxy Al) | | 5 |
| 7. | Benzalkonium chloride | | 0.1 |
| 8. | Siloxane of Synthesis Example 1 | | 15 |
| 9. | Glycerin | | 5 |
| 10. | BG | | 5 |
| 11. | Ethanol | | 5 |
| 12. | Phenoxyethanol | | 0.3 |
| 13. | Water | | 52.5 |
| | | Total | 00.0 |

| | | | |
|---|---|---|---|
| (*1) Mixture of 70-80% dimethicone + 20-30% (dimethicone/(PEG-10/15) crosspolymer (manufactured by Shin-Etsu Chemical Co., Ltd.) (*2) Mixture of 90-96% cyclopentasiloxane + 4-10% (dimethicone/vinyl dimethicone) crosspolymer (manufactured by Shin-Etsu Chemical Co., Ltd.) (*3) PEG-9 polydimethylsiloxyethyl dimethicone (manufactured by Shin-Etsu Chemical Co., Ltd.) | | | |

### [Production Method]

A: The ingredients 1 to 4 and 8 were uniformly mixed.
B: The ingredients 5 to 7 were uniformly mixed with the mixture obtained in A. The resultant mixture was uniformly dispersed and mixed using a mixer.
C: The ingredients 9 to 13 were dissolved.
D: The mixture obtained in C was added to the mixture obtained in B. The mixture was emulsified and was loaded into a container.

The deodorant cream obtained as described above was a W/O deodorant cream that had a dewy feeling on use, spread very smoothly and well, was free from an excessively dry or sticky feeling, and was excellent in long-lasting deodorizing effects.

### [Example 21] Hair oil

| | | Composition | % |
|---|---|---|---|
| 1. | Siloxane of Synthesis Example 2 | | 68 |
| 2. | Hydrogenated polyisobutene | | 10.3 |
| 3. | KF-7312J (*1) | | 3 |
| 4. | Dimethiconol | | 7 |
| 5. | Diethylhexyl succinate | | 10 |
| 6. | KF-9030 (*2) | | 1.5 |
| 7. | Tocopherol | | 0.1 |
| 8. | Fragrance | | 0.1 |
| | | Total | 100.0 |

| | | | |
|---|---|---|---|
| (*1) 50% Cyclopentasiloxane solution of trimethylsiloxysilicate (manufactured by Shin-Etsu Chemical Co., Ltd.) (*2) Dimethicone (gum solution) (manufactured by Shin-Etsu Chemical Co., Ltd.) | | | |

### [Production Method]

### A: The ingredients 1 to 8 were mixed uniformly to give a hair oil.

The hair oil obtained as described above spread lightly and gave gloss and smoothness to the hair.

The present invention is not limited to the embodiments described above, which are presented here for the purpose of illustration. Any embodiments having substantially the same constitution as the technical ideas set forth in the claims of the present invention and exhibiting similar working effects are encompassed within the technical scope of the present invention.

## Claims

1. A cosmetic comprising one or more branched organopolysiloxanes represented by formula (1) below:
(R¹₃SiO_{1/2})ₐ(R¹₂SiO_{2/2})_{b}(R¹SiO_{3/2})_{c}(SiO_{4/2})_{d} (1)
wherein R¹ is independently a hydrogen atom, a hydroxyl group, or a C1-C3 monovalent alkyl group, a is an integer of 0 to 5, b is an integer of 0 to 3, c is an integer of 0 to 2, d is 0 or 1, c + d is 1 or 2, and a + b + c + d is an integer of 4 to 7, two or more R¹ are 2 or C3 monovalent alkyl group,
the branched organopolysiloxanes having a boiling point at 10 hPa of 87 to 119°C.

2. The cosmetic according to claim 1, wherein the formula (1) has b = 0, d = 0, and c = 1.

3. The cosmetic according to claim 1 or 2, wherein the branched organopolysiloxane is one or more selected from 1,5-diethyl-3-trimethylsiloxy-1,1,3,5,5-pentamethyltrisiloxane and 1,5 -diethyl-3 -ethyldimethylsiloxy-1,1 ,3,5,5-pentamethyltrisiloxane.

4. The cosmetic according to any one of claims 1 to 3, further comprising an oil ingredient that is solid at 25°C.

5. The cosmetic according to claim 4, wherein the oil ingredient that is solid at 25°C is one or more selected from polyethylene, ceresin, ozokerite, candelilla wax, beeswax, microcrystalline wax, stearyl alcohol, behenyl alcohol, and cetanol.

6. The cosmetic according to any one of claims 1 to 5, further comprising an organic ultraviolet absorber.

7. The cosmetic according to claim 6, wherein the organic ultraviolet absorber is one or more selected from ethylhexyl methoxycinnamate, hexyl diethylaminohydroxybenzoylbenzoate, ethylhexyl salicylate, polysilicone-15, t-butylmethoxydibenzoylmethane, oxybenzone, methylenebisbenzotriazolyltetramethylbutylphenol, bisethylhexyloxyphenolmethoxyphenyltriazine, and octocrylene.
